(19) Europäisches Patentamt
European Patent Office
Office européen des brevets

(11) Veröffentlichungsnummer: **0 496 378 B1**

# (12) EUROPÄISCHE PATENTSCHRIFT

(45) Veröffentlichungstag der Patentschrift: **20.09.95**

(21) Anmeldenummer: **92101007.0**

(22) Anmeldetag: **22.01.92**

(51) Int. Cl.[6]: **C07C 257/18**, C07C 271/62, C07C 255/57, C07D 211/34, A61K 31/155

## (54) Biphenylderivate, diese Verbindungen enthaltende Arzneimittel und Verfahren zu ihrer Herstellung.

(30) Priorität: **24.01.91 DE 4102024**

(43) Veröffentlichungstag der Anmeldung:
**29.07.92 Patentblatt 92/31**

(45) Bekanntmachung des Hinweises auf die Patenterteilung:
**20.09.95 Patentblatt 95/38**

(84) Benannte Vertragsstaaten:
**AT BE CH DE DK ES FR GB GR IT LI LU NL PT SE**

(56) Entgegenhaltungen:
**EP-A- 0 048 433**
**DE-A- 3 215 676**
**US-A- 4 661 522**

(73) Patentinhaber: **Dr. Karl Thomae GmbH**

**D-88397 Biberach (DE)**

(72) Erfinder: **Himmelsbach, Frank, Dr. Dipl.-Chem.**
**Ahornweg 16**
**W-7951 Mittelbiberach (DE)**
Erfinder: **Pieper, Helmut, Dr. Dipl.-Chem.**
**Kapellenweg 5**
**W-7950 Biberach 1 (DE)**
Erfinder: **Austel, Volkhard, Dr. Dipl.-Chem.**
**Kapellenweg 7**
**W-7950 Biberach 1 (DE)**
Erfinder: **Linz, Günter, Dr. Dipl.-Chem.**
**Drosselweg 14**
**W-7951 Mittelbiberach (DE)**
Erfinder: **Müller, Thomas, Dr. Dipl.-Chem.**
**Gymnasiumstrasse 16**
**W-7951 Biberach 1 (DE)**
Erfinder: **Eisert, Wolfgang, Prof. Dr. Dr. Dr.**
**Hochmannweg 2**
**W-7950 Biberach 1 (DE)**
Erfinder: **Weisenberger, Johannes, Dr. Dipl.-Chem.**
**Haydnweg 5**
**W-7950 Biberach 1 (DE)**

EP 0 496 378 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Biphenylderivate der allgemeinen Formel

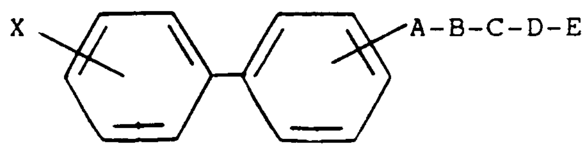

,(I)

deren Stereoisomere, einschließlich ihrer Gemische und deren Salze, insbesondere deren Physiologisch verträgliche Salze mit anorganischen oder organischen Säuren oder Basen, welche wertvolle Eigenschaften, insbesondere wertvolle pharmakologische Eigenschaften, aufweisen, vorzugsweise aggregationshemmende Wirkungen, diese Verbindungen enthaltende Arzneimittel und Verfahren zu ihrer Herstellung.

In der obigen allgemeinen Formel I bedeutet
mit der Maßgabe, daß der mit dem Rest X verbundene Phenylring durch ein Fluor-, Chlor- oder Bromatom und
der mit dem Rest A verbundene Phenylring durch ein Fluor- oder Chloratom, eine Hydroxy-, Methoxy-, Trifluormethyl-, Methylsulfenyl-, Methylsulfinyl-, Methylsulfonyl-, Nitro-, Amino-, Acetylamino-, Benzoylamino-, Methansulfonylamino- oder Benzolsulfonylaminogruppe oder durch eine oder zwei Methylgruppen oder durch ein oder zwei Bromatome substituiert sein kann,
X eine Aminomethyl-, Amidino- oder Guanidinogruppe, in denen ein Wasserstoffatom an einem der Stickstoffatome durch eine Alkylgruppe mit 1 bis 4 Kohlenstoffatomen, durch eine Methoxycarbonyl-, Ethoxycarbonyl- oder Benzyloxycarbonylgruppe ersetzt sein kann, oder eine Cyanogruppe,
A eine Bindung, ein Sauerstoff- oder Schwefelatom, eine -NH-CO-, $-NCH_3-CO-$, -CO-NH-, $-CO-NCH_3-$, $-NCH_3-$, -SO-, $-SO_2-$, $-SO_2-NH-$, $-SO_2-NCH_3-$, -CO-, -NH-CO-NH-, $-NH-SO_2-NH-$ oder $-NCH_3-CO-NCH_3-$Gruppe,
B eine Bindung, eine geradkettige oder verzweigte Alkylengruppen mit 1 bis 5 Kohlenstoffatomen, eine geradkettige oder verzweigte Alkenylengruppe mit 3 Kohlenstoffatomen, wobei die Doppelbindung nicht direkt mit einem Sauerstoff-, Schwefel- oder Phosphoratom der Reste A, C oder E verbunden sein kann, eine Cyclohexylen- oder Phenylengruppe,
C eine Bindung oder, sofern eine CO-Gruppe des Restes C nicht unmittelbar auf ein Sauerstoff- oder Schwefelatom oder eine -CO-NH- oder $-CO-NCH_3$-Gruppe des Restes A folgt, eine -CO-NH-, $-CO-NCH_3-$, $-CO-NH-(CH_2)_2-CH(CH_2-COOH)-$, $-CO-NCH_3-(CH_2)_2-CH(CH_2-COOH)-$, $-CO-NH-(CH_2)_2-CH(CH_2-COOCH_3)-$, $-CO-NCH_3-(CH_2)_2-CH(CH_2-COOCH_3)-$, Pyrrolidinylen-N-carbonyl-, Piperidinylen-N-carbonyl-, Piperazinylen-N-carbonyl- oder 4-Methanylyliden-piperidinocarbonyl-gruppe, wobei die Gruppe -D-E an die Methanylylidengruppe gebunden ist, eine 4-Hydroxy-4-piperidinylen-N-carbonyl-, 4-Carboxymethyl-4-piperidinylen-N-carbonyl- oder 4-Methoxycarbonylmethyl-4-piperidinylen-N-carbonyl-gruppe, wobei die Gruppe -D-E an die 4-Stellung gebunden ist, oder eine [[[2-(Methoxyphenyl)-ethyl]-aminocarbonyl]-methylen]-aminocarbonyl-Gruppe, wobei die Gruppe -D-E an das Methylenkohlenstoffatom gebunden ist,
D eine Bindung, eine geradkettige oder verzweigte Alkylengruppe mit 1 bis 3 Kohlenstoffatomen oder eine Alkylenphenylengruppe mit 1 oder 2 Kohlenstoffatomen im Alkylenteil und
E eine Carboxy-, Sulfo-, Phosphono-, 5-Tetrazolyl- oder O-Methyl-phophonogruppe, eine Alkoxcarbonylgruppe mit insgesamt 2 bis 5 Kohlenstoffatomen, in der der Alkoxyteil in 1- oder 2-Stellung durch eine Phenylgruppe substituiert sein kann, eine Aminocarbonyl-, Methylaminocarbonyl- oder Dimethylaminocarbonylgruppe, bedeuten, wobei mindestens einer der Reste A, B, C oder D keine Bindung darstellt, der Rest E nicht unmittelbar auf ein Heteroatom der Reste A oder C folgen kann, und, sofern X eine Aminomethylgruppe darstellt, der kürzeste Abstand zwischen der Aminogruppe und dem Rest E mindestens 12 Bindungen beträgt.

Bevozugte Verbindungen der obigen allgemeinen Formel I sind diejenigen, in denen
der mit dem Rest X verbundene Phenylring unsubstituiert ist und der mit dem Rest A verbundene Phenylring durch eine Hydroxy- oder Methoxygruppe substituiert sein kann,
X eine Aminomethyl- oder Amidinogruppe, in denen ein Wasserstoffatom an einem der Stickstoffatome durch eine Methoxycarbonyl-, Ethoxycarbonyl- oder Benzyloxycarbonylgruppe ersetzt sein kann,
A eine Bindung, ein Sauerstoffatom, eine -NH-CO-, -CO-NH-, $-CO-NCH_3-$, $-SO_2-NH-$ oder $-NH-SO_2-NH-$Gruppe,

B eine Bindung, eine geradkettige Alkylengruppe mit 1 bis 5 Kohlenstoffatomen, eine Cyclohexylen- oder Phenylengruppe,

C eine Bindung oder, sofern C nicht unmittelbar auf ein Sauerstoffatom oder eine -CO-NH- oder -CO-$NCH_3$-Gruppe des Restes A folgt, eine -CO-NH-Gruppe, eine Piperidinylen-N-carbonylgruppe, die in 3- oder 4-Stellung mit der Gruppe -D-E verknüpft ist, eine 4-Piperazinylen-N-carbonyl- oder 4-Methanylyliden-piperidinocarbonylgruppe, wobei die Gruppe -D-E an die Methanylylidengruppe gebunden ist, eine 4-Hydroxy-4-piperidinylen-N-carbonyl-, 4-Carboxymethyl-4-piperidinylen-N-carbonyl- oder 4-Methoxycarbonylmethylen-piperidinylen-N-carbonylgruppe, wobei der Rest -D-E an die 4-Stellung gebunden ist, oder eine [[-[2-(4-Methoxyphenyl)-ethyl]-aminocarbonyl]-methylen]-aminocarbonylgruppe, wobei die Gruppe -D-E an das Methylenkohlenstoffatom geknüpft ist,

D eine Bindung, eine Methylen-, Ethylen-, Methylenphenylen- oder Ethylenphenylengruppe und

E eine Carboxyl-, Methoxycarbonyl-, Ethoxycarbonyl-, Benzyloxycarbonyl-, Aminocarbonyl-, Dimethylaminocarbonyl- oder 5-Tetrazolylgruppe bedeuten, wobei mindestens einer der Reste A, B, C oder D keine Bindung darstellt und E nicht unmittelbar auf ein Heteroatom der Reste A oder C folgen kann, und, sofern X eine Aminomethylgruppe darstellt, der kürzeste Abstand zwischen der Aminogruppe und dem Rest E mindestens 12 Bindungen beträgt,

deren Stereoisomere, einschließlich ihrer Gemische und deren Salze.

Besonders bevozugte Verbindungen der obigen allgemeinen Formel I sind diejenigen, in denen

der Biphenylylrest unsubstituiert ist,

X eine Aminomethyl- oder Amidinogruppe, in denen ein Wasserstoffatom an einem der Stickstoffatome durch eine Methoxycarbonyl- oder Benzyloxycarbonylgruppe ersetzt sein kann,

A eine Bindung, ein Sauerstoffatom, eine -NH-CO- oder -CO-NH-Gruppe,

B eine Bindung, eine geradkettige Alkylengruppe mit 1 bis 5 Kohlenstoffatomen oder eine Cyclohexylengruppe,

C eine Bindung oder, sofern C nicht unmittelbar auf ein Heteroatom oder eine Carbonylgruppe des Restes A folgt, eine -CO-NH-Gruppe, eine Piperidinylen-N-carbonylgruppe, die in 3- oder 4-Stellung mit der Gruppe -D-E verknüpft ist, eine Piperazinylen-N-carbonylgruppe, wobei der Rest -D-E an die 4-Stellung gebunden ist, oder eine [[[2-(4-Methoxyphenyl)-ethyl]-aminocarbonyl]-methylen]-aminocarbonylgruppe, wobei die Gruppe -D-E an das Methylenkohlenstoffatom geknüpft ist,

D eine Bindung, eine Methylen- oder Ethylengruppe und E eine Carboxyl-, Methoxycarbonyl-, Ethoxycarbonyl- oder Benzyloxycarbonylgruppe bedeuten, wobei mindestens einer der Reste A, B, C oder D keine Bindung darstellt und E nicht unmittelbar auf ein Heteroatom der Reste A oder C folgen kann, und, sofern X eine Aminomethylgruppe darstellt, der kürzeste Abstand zwischen der Aminogruppe und dem Rest E mindestens 12 Bindungen beträgt,

deren Stereoisomere, einschließlich ihrer Gemische und deren Salze.

Als besonders bevorzugte Verbindungen seien beispielsweise folgende genannt:

4-Amidino-4'-[(4-carboxymethyl-piperidino)-carbonyl]-biphenyl,
4-Amidino-4'-[(4-carboxymethyl-piperazino)-carbonyl]-biphenyl,
4-Amidino-4'-[(4-carboxy-cyclohexyl)-aminocarbonyl]-biphenyl,
4-Amidino-4'-[(4-methoxycarbonylmethyl-piperidino)-carbonyl]-biphenyl,
4-Amidino-4'-[(4-methoxycarbonyl-cyclohexyl)-aminocarbonyl]-biphenyl,
4-(N-Methoxycarbonyl-amidino)-4'-[(4-methoxycarbonylmethylpiperidino)-carbonyl]-biphenyl,
4-Amidino-3'-[(4-carboxy-cyclohexyl)-aminocarbonyl]-4'-methoxy-biphenyl,
4-Aminomethyl-3'-[(4-carboxy-cyclohexyl)-aminocarbonyl]-4'-methoxy-biphenyl und
4-Amidino-3'-[N-(4-carboxy-cyclohexyl)-N-methyl-aminocarbonyl]-4'-hydroxy-biphenyl,

deren Stereoisomere, einschließlich ihrer Gemische und deren Salze.

Erfindungsgemäß erhält man die neuen Verbindungen der allgemeinen Formel I beispielsweise nach folgenden an und für sich bekannten Verfahren:

a) Zur Herstellung von Verbindungen der allgemeinen Formel I, in der X eine Amidinogruppe enthält:

Umsetzung einer gegebenenfalls im Reaktionsgemisch gebildeten Verbindung der allgemeinen Formel

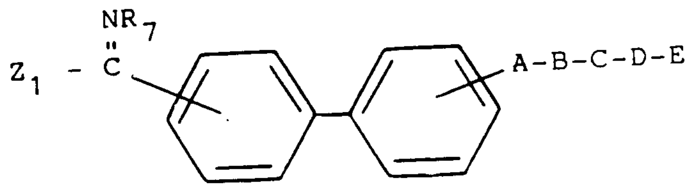
,(II)

in der
A, B, C, D und E wie eingangs definiert sind,
$R_7$ ein Wasserstoffatom oder eine Alkylgruppe mit 1 bis 4 Kohlenstoffatomen und
$Z_1$ eine Alkoxy- oder Aralkoxygruppe wie die Methoxy-, Ethoxy-, n-Propoxy-, Isopropoxy- oder Benzyloxygruppe oder eine Alkylthio- oder Aralkylthiogruppe wie die Methylthio-, Ethylthio-, n-Propylthio- oder Benzylthiogruppe oder eine Aminogruppe darstellen, mit einem Amin der allgemeinen Formel

$NH_2 - R_8$ ,(III)

in der
$R_8$ ein Wasserstoffatom, eine Alkylgruppe mit 1 bis 4 Kohlenstoffatomen, eine Hydroxygruppe, eine Alkoxygruppe mit 1 bis 3 Kohlenstoffatomen oder eine Aminogruppe bedeuten, oder mit deren Säureadditionssalzen.

Die Umsetzung wird zweckmäßigerweise in einem Lösungsmittel wie Methanol, Ethanol, n-Propanol, Wasser, Methanol/Wasser, Tetrahydrofuran oder Dioxan bei Temperaturen zwischen 0 und 150°C, vorzugsweise bei Temperaturen zwischen 20 und 120°C, mit einem entsprechenden freien Amin oder mit einem entsprechenden Säureadditionssalz wie beispielsweise Ammoniumcarbonat durchgeführt.

Eine Verbindung der allgemeinen Formel II erhält man beispielsweise durch Umsetzung eines entsprechenden Nitrils mit einem entsprechenden Alkohol wie Methanol, Ethanol, n-Propanol, Isopropanol oder Benzylalkohol in Gegenwart einer Säure wie Salzsäure oder durch Umsetzung eines entsprechenden Amids mit einem Trialkyloxoniumsalz wie Triethyloxonium-tetrafluorborat in einem Lösungsmittel wie Methylenchlorid, Tetrahydrofuran oder Dioxan bei Temperaturen zwischen 0 und 50°C, vorzugsweise jedoch bei 20°C, oder eines entsprechenden Nitrils mit Schwefelwasserstoff zweckmäßigerweise in einem Lösungsmittel wie Pyridin oder Dimethylformamid und in Gegenwart einer Base wie Triethylamin und anschließender Alkylierung des gebildeten Thioamids mit einem entsprechenden Alkyl- oder Aralkylhalogenid.

b) Zur Herstellung von Verbindungen der allgemeinen Formel I, in der X eine Aminomethylengruppe darstellt:

Reduktion einer Verbindung der allgemeinen Formel

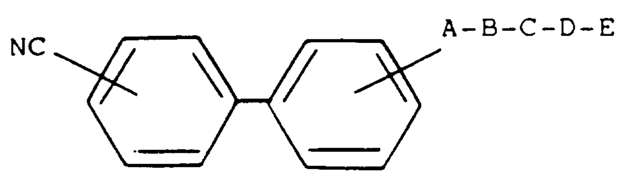
,(IV)

in der
A, B, C, D und E wie eingangs definiert sind.

Die Reduktion wird vorzugsweise in einem geeigneten Lösungsmittel wie Methanol, Methanol/Wasser, Methanol/Wasser/Ammoniak, Ethanol, Ether, Tetrahydrofuran, Dioxan oder Dimethylformamid in Gegenwart von katalytisch angeregtem Wasserstoff, z.B. von Wasserstoff in Gegenwart von Raney-Nickel, Platin oder Palladium/Kohle, oder in Gegenwart eines Metallhydrids wie Natriumborhydrid, Lithiumborhydrid oder

Lithiumaluminiumhydrid bei Temperaturen zwischen 0 und 100°C, vorzugsweise bei Temperaturen zwischen 20 und 80°C, durchgeführt.

c) Zur Herstellung von Verbindungen der allgemeinen Formel I, in der X eine Guanidinogruppe darstellt:

Umsetzung einer Verbindung der allgemeinen Formel

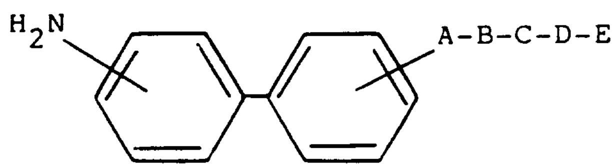

,(V)

in der
A, B, C, D und E wie eingangs definiert sind, oder dessen Säureadditionssalz mit Cyanamid.

Die Umsetzung wird zweckmäßigerweise in einem Lösungsmittel wie Dioxan, Dioxan/Wasser oder Tetrahydrofuran vorzugsweise bei Temperaturen zwischen 60 und 120°C, z.B. bei der Siedetemperatur des Reaktionsgemisches, durchgeführt.

d) Zur Herstellung von Verbindungen der allgemeinen Formel I, in der E eine Carboxylgruppe darstellt:

Umwandlung einer Verbindung der allgemeinen Formel

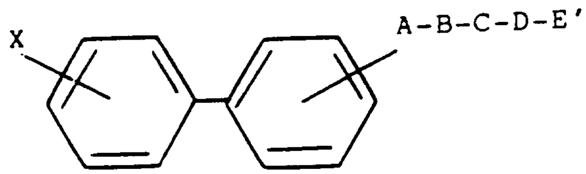

,(VI)

in der
A, B, C, D und X wie eingangs definiert sind und
E', das an ein Kohlenstoffatom gebunden ist, eine mittels Hydrolyse, Behandlung mit Säuren, Thermolyse oder Hydrogenolyse in eine Carboxyl- oder Bis(hydroxycarbonyl)methylgruppe überführbare Gruppe darstellt, und erforderlichenfalls anschließende Decarboxylierung.

Beispielsweise können funktionelle Derivate der Carboxylgruppe wie deren unsubstituierte oder substituierte Amide, Ester, Thioester, Trimethylsilylester, Orthoester, Iminoester, Amidine oder Anhydride, oder die Nitrilgruppe mittels Hydrolyse in eine Carboxylgruppe,
Ester mit tertiären Alkoholen, z.B. der tert. Butylester, mittels Behandlung mit einer Säure oder Thermolyse in eine Carboxylgruppe und
Ester mit Aralkanolen, z.B. der Benzylester, mittels Hydrogenolyse in eine Carboxylgruppe übergeführt werden.

Die Hydrolyse wird zweckmäßigerweise entweder in Gegenwart einer Säure wie Salzsäure, Schwefelsäure, Phosphorsäure, Trichloressigsäure oder Trifluoressigsäure, in Gegenwart einer Base wie Lithiumhydroxid, Natriumhydroxid oder Kaliumhydroxid in einem geeigneten Lösungsmittel wie Wasser, Wasser/Methanol, Ethanol, Wasser/Ethanol, Wasser/Isopropanol oder Wasser/Dioxan bei Temperaturen zwischen -10°C und 120°C, z.B. bei Temperaturen zwischen Raumtemperatur und der Siedetemperatur des Reaktionsgemisches, durchgeführt. Bei der Behandlung mit einer organischen Säure wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden.

Bedeutet E' in einer Verbindung der Formel VI eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen O und 50°C in die Carboxylgruppe übergeführt werden.

Bedeutet E' in einer Verbindung der Formel VI beispielsweise die tert. Butyloxycarbonylgruppe, so kann die tert. Butylgruppe auch durch Behandlung mit einer Säure wie Trifluoressigsäure, Ameisensäure, p-Toluolsulfonsäure, Schwefelsäure, Phosphorsäure oder Polyphosphorsäure gegebenenfalls in einem inerten Lösungsmittel wie Methylenchlorid, Chloroform, Benzol, Toluol, Tetrahydrofuran oder Dioxan vorzugsweise bei Temperaturen zwischen -10 °C und 120 °C, z.B. bei Temperaturen zwischen 0 und 60 °C, oder auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 °C und 100 °C, abgespalten werden.

Bedeutet E' in einer Verbindung der Formel VI beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Ethanol, Ethanol/Wasser, Eisessig, Essigsäureäthylester, Dioxan oder Dimethylformamid vorzugsweise bei Temperaturen zwischen 0 und 50 °C, z.B. bei Raumtemperatur, und einem Wasserstoffdruck von 1 bis 5 bar abgespalten werden. Bei der Hydrogenolyse können gleichzeitig andere Reste, z.B. eine Nitrogruppe zur Aminogruppe, oder eine Benzyloxygruppe zur Hydroxygruppe mitreduziert werden.

Die gegebenenfalls erforderliche anschließende Decarboxylierung wird vorzugsweise in einem Lösungsmittel wie Eisessig bei erhöhten Temperaturen, z.B. bei der Siedetemperatur des Reaktionsgemisches, durchgeführt.

e) Zur Herstellung von Verbindungen der allgemeinen Formel I, in der A eine $-NR_3-CO-$, $-CO-NR_3-$, $-SO_2-NR_3-$ oder $-NR_3-SO_2-$Gruppe darstellt:

Umsetzung einer Verbindung der allgemeinen Formel

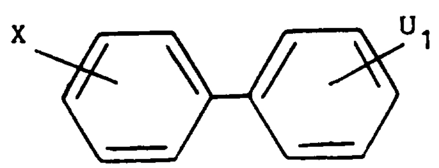

,(VII)

mit einer Verbindung der allgemeinen Formel

$U_2 - C - D - E$ ,(VIII)

in denen
C, D, E und X wie eingangs definiert sind,
einer der Reste $U_1$ oder $U_2$ eine $HNR_3$-Gruppe, wobei $R_3$ wie eingangs definiert ist, und
der andere der Reste $U_1$ oder $U_2$ eine $Z_2$-A'-Gruppe bedeuten, wobei
A' eine Carbonyl- oder Sulfonylgruppe und
$Z_2$ eine Hydroxygruppe oder eine nukleophile Austrittsgruppe wie ein Halogenatom, eine Alkoxy-, Aralkoxy-, Aryloxy-, Alkylthio-, Arylthio- oder Alkoxycarbonyloxygruppe darstellen, z.B. ein Chlor-, Brom- oder Jodatom, eine Methoxy-, Ethoxy-, Benzyloxy-, Phenoxy-, Methylthio-, Phenylthio- oder Isobutyloxycarbonyloxygruppe,
oder mit deren reaktionsfähigen Derivaten wie deren inneren Anhydriden.

Die Umsetzung wird zweckmäßigerweise in einem Lösungsmittel wie Methanol, Methylenchlorid, Chloroform, Tetrachlorkohlenstoff, Ether, Tetrahydrofuran, Dioxan, Benzol, Toluol, Acetonitril, Dimethylsulfoxid, Sulfolan oder Dimethylformamid gegebenenfalls in Gegenwart einer anorganischen oder organischen Base, gegebenenfalls in Gegenwart eines Reaktionsbeschleunigers wie 4-Dimethylamino-pyridin, Kupfer oder Kupfer(I)chlorid oder auch, wenn $Z_2$ eine Hydroxygruppe darstellt, gegebenenfalls in Gegenwart eines die Säure aktivierenden Mittels wie N,N'-Carbonyldiimidazol- oder N,N'-Dicyclohexylcarbodiimid gegebenenfalls in Gegenwart von Hydroxybenztriazol oder Hydroxysuccinimid oder gegebenenfalls in Gegenwart eines wasserentziehenden Mittels oder gegebenenfalls in Gegenwart eines die Aminogruppe aktivierenden Mittels bei Temperaturen zwischen -20 und 200 °C, vorzugsweise jedoch bei Temperaturen zwischen -10

und 160 °C, durchgeführt.

f) Zur Herstellung von Verbindungen der allgemeinen Formel I, in der die E-D-C-Gruppe eine HOOC-$(CH_2)_n$-CO-NH-Gruppe darstellt:

Hydrolyse einer Verbindung der allgemeinen Formel

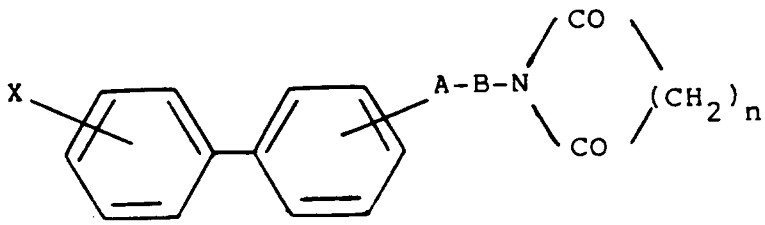

,(IX)

in der
A, B, X und n wie eingangs definiert sind.

Die Hydrolyse wird zweckmäßigerweise in Gegenwart einer Base wie Natriumhydroxid oder Kaliumhydroxid in einem geeigneten Lösungsmittel wie Wasser, Wasser/Methanol, Ethanol, Wasser/Ethanol, Wasser/Isopropanol oder Wasser/Dioxan bei Temperaturen zwischen -10 °C und 120 °C, z.B. bei Temperaturen zwischen Raumtemperatur und der Siedetemperatur des Reaktionsgemisches, durchgeführt.

g) Zur Herstellung von Verbindungen der allgemeinen Formel I, in der E eine Alkoxcarbonylgruppe mit insgesamt 2 bis 6 Kohlenstoffatomen, in der der Alkoxyteil in 1-, 2- oder 3-Stellung durch eine Aryl- oder Pyridylgruppe oder in 2- oder 3-Stellung durch eine Pyrrolidino-, Piperidino-, Hexamethylenimino-, 2-Oxo-1-pyrrolidinyl-, Morpholino-, Thiomorpholino- oder 1,1-Dioxido-thiomorpholinogruppe oder durch eine gegebenenfalls in 4-Stellung durch einen Rest $R_5$ substituierte Piperazinogruppe substituiert sein kann, darstellt:

Umsetzung einer Verbindung der allgemeinen Formel

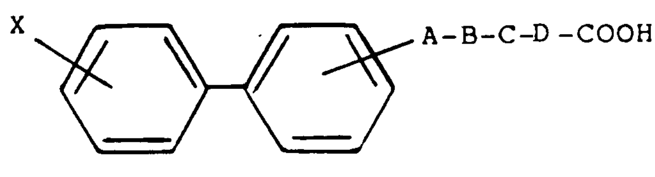

,(X)

in der
A, B, C, D und X wie eingangs definiert sind, oder deren reaktionsfähige Derivate wie deren Ester, Anhydride oder Halogenide, mit einer Verbindung der allgemeinen Formel

H - $R_9$ ,(XI)

in der
$R_9$ eine Alkoxygruppe mit 1 bis 5 Kohlenstoffatomen, in der der Alkoxyteil in 1-, 2- oder 3-Stellung durch eine Aryl- oder Pyridylgruppe oder in 2- oder 3-Stellung durch eine Pyrrolidino-, Piperidino-, Hexamethylenimino-, 2-Oxo-1-pyrrolidinyl-, Morpholino-, Thiomorpholino- oder 1,1-Dioxido-thiomorpholinogruppe oder durch eine gegebenenfalls in 4-Stellung durch einen Rest $R_5$ substituierte Piperazinogruppe substituiert sein kann, darstellt.

Die Umsetzung wird zweckmäßigerweise in einem Lösungsmittel wie Tetrahydrofuran, Chloroform, Dimethylformamid oder in einem entsprechenden Alkohol in Gegenwart eines die Säure aktivierenden Mittels oder eines wasserentziehenden Mittels, z.B. in Gegenwart von Chlorameisensäureisobutylester, Thionylchlorid, Trimethylchlorsilan, Salzsäure, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer Base wie Natriumcarbonat, Kaliumcarbonat, Kalium-tert.butylat oder 1-Hydroxy-benztriazol/Triethylamin oder in Gegenwart einer tertiären organischen Base wie Triethylamin, N-Ethyl-diisopropylamin, N-Methyl-morpholin oder Pyridin, welche gleichzeitig als Lösungsmittel dienen können, bei Temperaturen zwischen -30 und 100°C, vorzugsweise jedoch bei Temperaturen zwischen -10 und 80°C, durchgeführt. Die Umsetzung kann jedoch auch mit einem entsprechenden Säurehalogenid oder Säureanhydrid gegebenenfalls in Gegenwart eines säurebindenden Mittels wie vorstehend beschrieben durchgeführt werden.

h) Zur Herstellung von Verbindungen der allgemeinen Formel I, in der die Gruppe X eine Cyano-, Alkoxycarbonyl- oder Aralkoxycarbonylgruppe enthält:

Umsetzung einer Verbindung der allgemeinen Formel

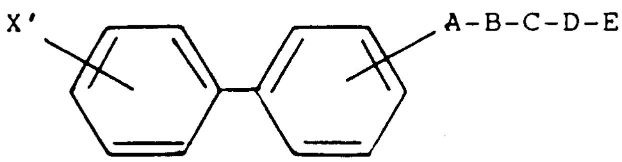

,(XII)

in der
A, B, C, D und E wie eingangs definiert sind und
X' eine Amino-, Aminoalkyl-, Amidino-, Guanidino- oder Guanidinoalkylgruppe darstellt, mit Bromcyan oder mit einem Ester der allgemeinen Formel

$Z_3$ - CO - $OR_3$'  ,(XIII)

in der
$R_3$' eine gegebenenfalls durch eine Phenylgruppe substituierte Alkylgruppe mit 1 bis 3 Kohlenstoffatomen und
$Z_3$ eine nukleophile Austrittsgruppe wie ein Halogenatom, eine Azidogruppe, eine Alkoxycarbonyloxy-, Aralkoxycarbonyloxy- oder Aryloxygruppe, z.B. ein Chlor- oder Bromatom, eine Methoxycarbonyloxy-, Ethoxycarbonyloxy-, Benzyloxycarbonyloxy- oder Nitrophenyloxygruppe, darstellen.

Die Umsetzung wird zweckmäßigerweise in einem Lösungsmittel wie Tetrahydrofuran, Chloroform, Dimethylformamid, Dioxan, Methylenchlorid oder Diethylether gegebenenfalls in Gegenwart einer Base wie Natriumcarbonat, Kaliumcarbonat, Kalium-tert.-butylat oder 1-Hydroxy-benztriazol/Triethylamin oder in Gegenwart einer tertiären organischen Base wie Triethylamin, N-Ethyl-diisopropylamin, N-Methyl-morpholin, 4-Dimethylaminopyridin oder Pyridin, welche gleichzeitig als Lösungsmittel dienen können, bei Temperaturen zwischen -30 und 100°C, vorzugsweise jedoch bei Temperaturen zwischen -10 und 80°C, durchgeführt.

i) Zur Herstellung von Verbindungen der allgemeinen Formel I, in der A oder C eine Sulfinyl- oder Sulfonylgruppe oder $R_1$ oder $R_2$ eine Alkylsulfinyl- oder Alkylsulfonylgruppe darstellen oder E eine 1-Oxidothiomorpholino- oder 1,1-Dioxidothiomorpholinogruppe enthält:

Oxidation einer Verbindung der allgemeinen Formel

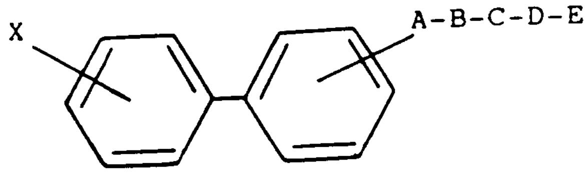

,(XIV)

in der
A, B, C, D, E und X mit der Maßgabe wie eingangs definiert sind, daß A oder C ein Schwefelatom oder eine Sulfinylgruppe darstellt oder $R_1$ oder $R_2$ eine Sulfenyl- oder Sulfinylgruppe oder E eine Thiomorpholino- oder 1-Oxidothiomorpholinogruppe enthalten.

Die Oxidation wird vorzugsweise in einem Lösungsmittel oder Lösungsmittelgemisch, z.B. in Wasser, Wasser/Pyridin, Aceton, Eisessig, Methylenchlorid, Eisessig/Acetanhydrid, verdünnter Schwefelsäure oder Trifluoressigsäure, je nach dem verwendeten Oxidationsmittel zweckmäßigerweise bei Temperaturen zwischen -80 und 100°C durchgeführt.

Zur Herstellung einer entsprechenden S-Oxidverbindung der allgemeinen Formel I wird die Oxidation zweckmäßigerweise mit einem Äquivalent des verwendeten Oxidationsmittels durchgeführt, z.B. mit Wasserstoffperoxid in Eisessig, Trifluoressigsäure oder Ameisensäure bei 0 bis 20°C oder in Aceton bei 0 bis 60°C, mit einer Persäure wie Perameisensäure in Eisessig oder Trifluoressigsäure bei 0 bis 50°C oder mit m-Chlorperbenzoesäure in Methylenchlorid oder Chloroform bei -20 bis 60°C, mit Natriummetaperjodat in wäßrigem Methanol oder Ethanol bei -15 bis 25°C, mit Brom in Eisessig oder wäßriger Essigsäure, mit N-Brom-succinimid in Ethanol, mit tert.Butylhypochlorit in Methanol bei -80 bis -30°C, mit Jodbenzodichlorid in wäßrigem Pyridin bei 0 bis 50°C, mit Salpetersäure in Eisessig bei 0 bis 20°C, mit Chromsäure in Eisessig oder in Aceton bei 0 bis 20°C und mit Sulfurylchlorid in Methylenchlorid bei -70°C, der hierbei erhaltene ThioätherChlor-Komplex wird zweckmäßigerweise mit wäßrigem Ethanol hydrolysiert.

Zur Herstellung einer S,S-Dioxidverbindung der allgemeinen Formel I wird die Oxidation zweckmäßigerweise mit einem bzw. mit zwei oder mehr Äquivalenten des verwendeten Oxidationsmittels durchgeführt, z.B. mit Wasserstoffperoxid in Eisessig/Acetanhydrid, Trifluoressigsäure oder in Ameisensäure bei 20 bis 100°C oder in Aceton bei 0 bis 60°C, mit einer Persäure wie Perameisensäure oder m-Chlorperbenzoesäure in Eisessig, Trifluoressigsäure, Methylenchlorid oder Chloroform bei Temperaturen zwischen 0 und 60°C, mit Salpetersäure in Eisessig bei 0 bis 20°C, mit Chromsäure oder Kaliumpermanganat in Eisessig, Wasser/Schwefelsäure oder in Aceton bei 0 bis 20°C.

Bei den vorstehend beschriebenen Umsetzungen a) bis i) können gegebenenfalls vorhandene reaktive Gruppen wie Hydroxy-, Carboxy-, Amino- oder Alkylaminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, tert.Butyl-, Trityl-, Benzyl- oder Tetrahydropyranylgruppe,
als Schutzreste für eine Carboxylgruppe die Trimethylsilyl-, Methyl-, Ethyl-, tert.Butyl-, Benzyl- oder Tetrahydropyranylgruppe und
als Schutzrest für eine Amino-, Alkylamino- oder Iminogruppe die Acetyl-, Benzoyl-, Ethoxycarbonyl-, tert.Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder mittels Etherspaltung, z.B. in Gegenwart von Jodtrimethylsilan, bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur, erfolgen.

Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel I mit mindestes 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)- oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxylgruppe enthalten, gewünschtenfalls anschließend in ihre Additionssalze mit anorganischen oder organischen Basen, insbesondere für die pharmazeutische Anwendung in ihre physiologisch verträglichen Additionssalze, überführen. Als Basen kommen hierbei beispielsweise Natriumhydroxid, Kaliumhydroxid, Cyclohexylamin, Ethanolamin, Diethanolamin und Triethanolamin in Betracht.

Die als Ausgangsstoffe verwendeten Verbindungen der Formeln II bis XIV sind teilweise literaturbekannt oder man erhält diese nach literaturbekannten Verfahren (siehe Beispiele). Außerdem werden diese teilweise in unserer nicht vorveröffentlichten Deutschen Patentanmeldung P 40 35 961.1 vom 2. November 1990 beschrieben.

Wie bereits eingangs erwähnt, weisen die neuen Biphenylderivate der allgemeinen Formel I und deren Additionssalze, insbesondere deren physiologisch verträgliche Additionssalze mit anorganischen oder organischen Säuren oder Basen, wertvolle Eigenschaften auf. So weisen die neuen Verbindungen der allgemeinen Formel I, in denen X eine gegebenenfalls substituierte Amino-, Amidino- oder Guanidinogruppe oder eine gegebenenfalls in vivo in eine gegebenenfalls substituierte Amino-, Amidino- oder Guanidinogruppe überführbare Gruppe, z.B. eine durch eine Alkoxycarbonylgruppe substituierte Amino-, Amidino- oder Guanidinogruppe, enthält und -C-D-E Carboxyl-, Sulfo-, Phosphono-, O-Alkyl-phosphono- oder 5-Tetrazolylgruppen oder in vivo in eine Carboxyl-, Sulfo-, Phosphono-, O-Alkyl-phosphono- oder Tetrazolylgruppen überführbare Gruppen, z.B. durch eine Alkoxygruppe substituierte Carbonylgruppen, enthält, wertvolle pharmakologische Eigenschaften auf, neben einer entzündungshemmenden und den Knochenabbau hemmenden Wirkung insbesondere antithrombotische, antiaggregatorische und tumor- bzw. metastasenhemmende Wirkungen.

Die Verbindungen der allgemeinen Formel I, in der X eine Cyanogruppe darstellt, stellten wertvolle Zwischenprodukte zur Herstellung der entsprechenden Aminomethyl- und Amidinoverbindungen der allgemeinen Formel I dar.

Beispielsweise wurden die Verbindungen der allgemeinen Formel I auf ihre biologischen Wirkungen wie folgt untersucht:

Fibrinogen-Bindung an Humanthrombozyten

Das durch Punktion einer Antekubitalvene gewonnene Blut wird mit Trinatriumcitrat (Endkonzentration: 13 mM) antikoaguliert und 10 Minuten bei 170 *g zentrifugiert. Das überstehende plättchenreiche Plasma wird auf eine Sepharose 2B-Säule (Pharmacia) gegeben und mit einer Lösung aus 90 mM Kochsalz, 14 mM Trinatriumcitrat, 5 mM Glucose und 50 mM Tris(hydroxymethyl)aminomethan, eingestellt auf pH 7,4, eluiert. Die vor den Plasmaproteinen erscheinenden gelfiltrierten Plättchen (GFP) werden für die Bindungsversuche verwendet.

50 µl einer 60 mM Calziumchlorid-Lösung, 50 µl einer 0,6 mM Adenosindiphosphat-Lösung, 100 µl Substanzlösung bzw. Lösungsmittel und 50 µl Fibrinogenlösung (enthaltend 3 µg 125-J-Fibrinogen) werden zu 750 µl GFP gegeben und bei Raumtemperatur 20 Minuten inkubiert. Die unspezifische Bindung wird in Gegenwart von 3 mg/ml kaltem Fibrinogen bestimmt.

900 µl des Inkubates werden vorsichtig auf 250 µl Silikonöl (AP 38: AR 20, 1:2 v/v, Wacker Chemie) in Eppendorf-Gefäße pipettiert und 2 Minuten bei 10 000 *g zentrifugiert. Der wäßrige Überstand und ein Teil des Öls werden abgezogen, die Gefäßspitze mit dem Plättchenpellet abgeschnitten und im Gamma-Zähler die Menge des gebundenen Fibrinogens bestimmt. Aus einer Konzentrationsreihe wird die Substanzkonzentration ermittelt, welche die Fibrinogenbindung zu 50 % hemmt und als $IC_{50}$ angegeben.

2. Antithrombotische Wirkung

Methodik: Die Thrombozytenaggregation wird nach der Methode von Born und Cross (J. Physiol. 170, 397 (1964)) in plättchenreichem Plasma gesunder Versuchspersonen gemessen. Zur Gerinnungshemmung wird das Blut mit Natriumcitrat 3,14 % im Volumenverhältnis 1:10 versetzt.

Collagen-induzierte Aggregation: Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München.

Vor der Collagen-Zugabe wird das Plasma jeweils 10 Minuten mit der Substanz bei 37 °C inkubiert.

Aus den erhaltenen Meßzahlen wird graphisch eine $EC_{50}$ bestimmt, die sich auf eine 50%ige Änderung der "optical density" im Sinne einer Aggregationshemmung bezieht.

Die nachfolgende Tabelle enthält die gefundenen Ergebnisse:

| Substanz (Beispiel Nr.) | Fibrinogen-Bindungstest $IC_{50}$[nM] | Hemmung der Plättchenaggregation $EC_{50}$[nM] |
|---|---|---|
| 1 | 290 | 1 100 |
| 1(1) | 160 | 1 100 |
| 1(2) | 120 | 7 000 |
| 1(3) | 1 800 | 13 000 |
| 1(4) | 350 | 1 700 |
| 1(5) | 65 000 | >100 000 |
| 1(6) | 330 | 1 200 |
| 1(7) | 1 900 | 3 800 |
| 1(39) | 24 | 100 |
| 1(41) | 520 | 2 600 |
| 1(45) | 220 | 2 000 |
| 1(47) | 470 | 2 700 |
| 1(65) | 220 | 2 400 |
| 1(67) | 180 | 350 |
| 1(68) | 560 | 3 400 |
| 1(69) | 3 100 | 12 000 |
| 1(74) | 2 700 | 10 700 |
| 1(80) | 31 | 40 |
| 1(94) | | 40 |
| 2 | 210 | 10 000 |
| 2(1) | >10 000 | 56 000 |
| 2(2) | 46 | 45 000 |
| 2(11) | 360 | 2 200 |
| 3 | 1 300 | 16 000 |
| 6 | 570 | 2 600 |
| 6(1) | 5 600 | 3 700 |
| 6(2) | 14 000 | 40 000 |
| 6(3) | 18 000 | 17 000 |
| 6(4) | 47 000 | 4 400 |
| 6(8) | 5 200 | 3 400 |

| Substanz (Beispiel Nr.) | Fibrinogen-Bindungstest $IC_{50}$[nM] | Hemmung der Plättchenaggregation $EC_{50}$[nM] |
|---|---|---|
| 6(9) | 970 | 1 300 |
| 6(46) | 19 000 | 82 000 |
| 6(47) | 4 900 | 42 000 |
| 6(48) | 25 000 | 590 |
| 6(49) | 32 000 | 36 000 |
| 6(50) | 340 | 890 |
| 6(52) | 16 000 | 38 000 |
| 6(56) | 5 900 | 4 900 |
| 6(58) | 34 000 | 23 000 |
| 6(72) | 7 400 | 3 400 |
| 6(74) | 24 000 | 3 200 |
| 6(75) | 27 000 | 3 200 |
| 6(81) | 25 000 | 34 000 |
| 6(86) | 5 700 | 21 000 |
| 6(87) | 3 800 | 60 |
| 6(98) | | 370 |
| 8(7) | 59 000 | >10 000 |
| 10(1) | 800 | 1 800 |

Außerdem hemmt beispielsweise die Verbindung des Beispiels 8(5) die durch Collagen induzierte Thrombocytenaggregation ex vivo am Rheususaffen nach oraler Gabe von 1 mg/kg länger als 8 Stunden.

Die neuen Verbindungen sind gut verträglich, da beispielsweise nach intravenöser Gabe von 60 mg/kg der Verbindung des Beispiels 1(39) an der Maus keines der drei getesteten Tiere verstarb. Ähnliche Resultate ergaben die Verbindungen der Beispiele 1, 1(1) und 2(11) bei einer Dosis von 30 mg/kg, wobei während der Injektionsphase mit den Verbindungen der Beispiele 1(1) und 2(11) 1 bzw. 2 Tiere sediert waren. Ferner wurden bei peroraler Gabe von 2,0 g/kg der Verbindung des Beispiels 8(5) weder an der Ratte noch an der Maus toxische Effekte beobachtet.

Auf Grund ihrer Hemmwirkung auf Zell-Zell- bzw. Zell-Matrix-Wechselwirkungen eignen sich die neuen cyclischen Iminoderivate der allgemeinen Formel I und ihre physiologisch verträglichen Additionssalze zur Bekämpfung bzw. Verhütung von Krankheiten, bei denen kleinere oder größere Zell-Aggregate auftreten oder Zell-Matrixinteraktionen eine Rolle spielen, z.B. bei der Bekämpfung bzw. Verhütung von venösen und arteriellen Thrombosen, von zerebrovasculären Erkrankungen, von Lungenembolien, des Herzinfarktes, der Arteriosklerose, der Osteoporose und der Metastasierung von Tumoren. Weiterhin eignen sich diese zur Begleittherapie bei der Thrombolyse mit Fibrinolytica oder Gefäßinterventionen wie transluminaler Angioplastie oder auch bei der Therapie von Schockzuständen, des Diabetes und von Entzündungen.

Für die Bekämpfung bzw. Verhütung der vorstehend erwähnten Krankheiten liegt die Dosis zwischen 0,1 µg und 20 mg/kg Körpergewicht, vorzugsweise bei 1 µg bis 10 mg/kg Körpergewicht, bei bis zu 4 Gaben pro Tag. Hierzu lassen sich die erfindungsgemäß hergestellten Verbindungen der Formel I, gegebenenfalls in Kombination mit anderen Wirksubstanzen, zusammen mit einem oder mehreren inerten

üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Stearylalkohol, Carboxymethylcellulose oder fetthaltigen Substanze wie Hartfett oder deren geeigneten Gemischen, in übliche galenische Zubereitungen wie Tabletten, Dragées, Kapseln, Pulver, Suspensionen, Lösungen, Sprays oder Zäpfchen einarbeiten.

Die nachfolgenden Beispiele sollen die Erfindung näher erläutern:

Beispiel I

4'-Cyano-biphenylyl-4-essigsäure

Eine Mischung aus 11,3 g 4'-Brom-biphenylyl-4-essigsäure (Schmelzpunkt: 172-175 °C, hergestellt aus 4-Acetyl-4'-brom-biphenyl durch Behandeln mit Morpholin und Schwefel und anschließende Hydrolyse mit Kaliumhydroxid), 3,48 g Kupfer-(I)-cyanid und 100 ml Dimethylformamid wird 12 Stunden zum Rückfluß erhitzt und nach dem Abkühlen eingeengt. Der Rückstand wird zwischen 1n Natronlauge und Methylenchlorid, dem etwas Methanol zugesetzt wird, verteilt. Die wäßrige Phase wird angesäuert und mit Methylenchlorid extrahiert. Die Methylenchloridphase wird mit Aktivkohle behandelt, eingeengt, der feste Rückstand mit einer Mischung aus Ether und Petrolether verrieben und abfiltriert.
Ausbeute: 5,1 g (55 % der Theorie),
$R_f$-Wert: 0,46 (Kieselgel; Methylenchlorid/Ethanol = 9:1)
Analog wird folgende Verbindung erhalten:

(1) 4-Cyano-biphenylyl-4'-carbonsäure-methylester
Schmelzpunkt: 140-142 °C
Die erforderliche Ausgangsverbindung 4-Brom-4'-biphenylyl-carbonsäure-methylester (Schmelzpunkt: 140-142 °C) erhält man durch Veresterung der Säure mit methanolischer Salzsäure. 4-Brom-4'-biphenylyl-carbonsäure erhält man durch Umsetzung von 4-Acetyl-4'-brom-biphenyl mit Brom und Natronlauge.

Beispiel II

4-Cyano-4'-(2-hydroxyethyl)-biphenyl

20,5 g 4'-Cyano-biphenylyl-4-essigsäuremethylester werden in 350 ml Tetrahydrofuran gelöst. Man gibt unter Rühren 1,8 g Lithiumborhydrid zu und rührt zwei Tage bei Raumtemperatur. Man destilliert das Lösungsmittel ab, versetzt mit Wasser und filtriert den gebildeten Niederschlag ab. Er wird neutral gewaschen und ohne weitere Reinigung weiter verwendet.
Ausbeute: 17,2 g (94 % der Theorie)
$R_f$-Wert: 0,38 (Kieselgel; Methylenchlorid)
Analog wird folgende Verbindung erhalten:

(1) 4-Cyano-4'-hydroxymethyl-biphenyl

Beispiel III

4-(2-Brom-ethyl)-4'-cyano-biphenyl

Eine Mischung aus 17,2 g 4-Cyano-4'-(2-hydroxyethyl)-biphenyl, 6,8 ml Pyridin und 75 ml Methylenchlorid wird auf -5 °C abgekühlt und dazu 7,8 ml Thionylbromid unter Rühren getropft. Man läßt auf Raumtemperatur kommen, erhitzt nach 2 Stunden noch eine Stunde auf 45 °C und läßt über Nacht bei Raumtemperatur stehen. Die Methylenchloridphase wird mit Eiswasser bis zum Verschwinden der stark sauren Reaktion gewaschen, filtriert und eingeengt. Der Rückstand wird ohne weitere Reinigung weiter verwendet.
Ausbeute: 20,4 g (93 % der Theorie)
$R_f$-Wert: 0,60 (Kieselgel; Methylenchlorid)
Analog wird folgende Verbindung erhalten:

(1) 4-Brommethyl-4'-cyano-biphenyl

Beispiel IV

2-[(2-Ethoxycarbonyl-ethyl)-aminosulfonyloxy]-phenol

1,54 g β-Alanin-ethylester-hydrochlorid werden unter Zusatz von 1,55 g N-Ethyl-diisopropylamin in 10 ml Dimethylformamid gelöst und unter Eiskühlung mit 1,9 g Benzo-dioxathiazol-2,2-dioxid versetzt. Man rührt 2 Stunden bei Raumtemperatur, zieht das Dimethylformamid im Vakuum ab (Badtemperatur maximal 30 °C) und reinigt den Rückstand durch Chromatographie an Kieselgel (Elutionsmittel: Cyclohexan/Essigester = 8:2).
Ausbeute: 1,4 g (48 % der Theorie),
$R_f$-Wert: 0,31 (Kieselgel; Cyclohexan/Essigester = 7:3)

Beispiel V

3-Carboxy-4'-cyano-4-hydroxy-biphenyl

Zu einer 30 Minuten bei -20 °C gerührten Mischung aus 25 ml Oxalylchlorid, 50 ml Methylenchlorid und 25 g Aluminiumchlorid wird eine Lösung von 12 g 4-Cyano-4'-methoxy-biphenyl in 75 ml Methylenchlorid getropft. Man rührt 5 Stunden bei -20 °C, läßt unter anfänglicher Eiskühlung im Verlauf von 16 Stunden auf Raumtemperatur kommen und rührt dann noch weitere 3 Stunden. Man gießt auf Eiswasser, rührt 30 Minuten nach, extrahiert mit Essigester und dampft die Essigesterphase bis zur Kristallisation ein.
Ausbeute: 10,3 g (75 % der Theorie),
Schmelzpunkt: 244-246 °C

Beispiel VI

4-(Carboxymethyloxy)-4'-cyano-biphenyl

32,9 g 4-(tert.Butyloxycarbonylmethyloxy)-4'-cyano-biphenyl werden in 250 ml Methylenchlorid gelöst und langsam mit 137 ml Trifluoressigsäure versetzt. Man rührt 3 Stunden bei Raumtemperatur, dampft zur Trockene ein und verreibt den Rückstand mit Wasser.
Ausbeute: 26,3 g (98 % der Theorie),
Schmelzpunkt: 202-204 °C

Beispiel VII

4-(tert.Butyloxycarbonylmethyloxy)-4'-cyano-biphenyl

Hergestellt analog Beispiel 13 aus 4-Cyano-4'-hydroxy-biphenyl und Bromessigsäure-tert.butylester.
Schmelzpunkt: 110-112 °C

Beispiel VIII

3-Chlorsulfonyl-4'-cyano-4-methoxy-biphenyl

Hergestellt aus 6,1 g Natrium-4'-cyano-4-methoxy-3-biphenylyl-sulfonat durch 3,5-stündiges Rückflußkochen mit 30 ml Phosphoroxychlorid und Einrühren in Wasser.
Ausbeute: 4,7 g (81 % der Theorie)
$R_f$-Wert: 0,29 (Kieselgel; Cyclohexan/Essigester = 2:1)
Das Ausgangsmaterial erhält man durch Sulfonierung von 4-Cyano-4'-methoxy-biphenyl mit Chlorsulfonsäure.

Herstellung der Endverbindungen:

Beispiel 1

4-Amidino-4'-[[(2-carboxy-ethyl)-aminocarbonyl]-methyl]-biphenyl

0,5 g 4-Amidino-4'-[[(2-methoxycarbonyl-ethyl)-aminocarbonyl]-methyl]-biphenyl werden 16 Stunden mit 10 ml 1n Natronlauge bei Raumtemperatur gerührt. Man versetzt mit 0,7 g Ammoniumchlorid, engt ein und verrührt den Rückstand mit Wasser. Das gebildete Kristallisat wird abgesaugt und mit Aceton gewaschen.
Ausbeute: 0,41 g (97 % der Theorie),
Schmelzpunkt: über 250 ° C
$R_f$-Wert: 0,04 (Kieselgel; Methylenchlorid/Ethanol/konzentriertes Ammoniak = 4:1:0,25)
Analog werden folgende Verbindungen erhalten:
(1) 4-Amidino-4'-(4-carboxybutyrylamino)-biphenyl
(Zur Verseifung wird ein Gemisch aus gleichen Teilen 1n Natronlauge und Methanol eingesetzt)
Schmelzpunkt: über 240 ° C
$R_f$-Wert: 0,50 (Kieselgel; Butanol/Eisessig/Wasser = 3:1:1)
(2) 4-Amidino-4'-[(3-carboxy-propyl)-aminocarbonyl]-biphenyl
Schmelzpunkt: über 260 ° C
(Zur Verseifung wird Lithiumhydroxid eingesetzt)
$R_f$-Wert: 0,06 (Kieselgel; Methylenchlord/Ethanol/konzentriertes Ammoniak = 4:1:0,25)
(3) 4-Amidino-4'-[[(carboxymethylamino)-carbonyl]-methoxy]-biphenyl
Schmelzpunkt: 285 ° C (Zers.)

| | | | | | | |
|---|---|---|---|---|---|---|
| Ber. (x 0,5 $H_2O$):<br>Gef.: | C | 60,71<br>60,36 | H | 5,39<br>5,27 | N | 12,49<br>12,01 |

(4) 4-Amidino-4'-[[(2-carboxy-ethyl)-aminocarbonyl]-methyloxy]-biphenyl
Schmelzpunkt: 290 ° C (Zers.)

| | | | | | | |
|---|---|---|---|---|---|---|
| Ber. (x 0,75 $H_2O$):<br>Gef.: | C | 60,91<br>61,38 | H | 5,78<br>5,88 | N | 11,84<br>11,55 |

(5) 4-Amidino-4'-[[(3-carboxymethyl-phenyl)-aminocarbonyl]-methyl]-biphenyl
(Zur Verseifung wird Lithiumhydroxid eingesetzt)
Schmelzpunkt: 262-264 ° C
(6) 4-Amidino-4'-[[(4-carboxy-piperidino)-carbonyl]-methyloxy]-biphenyl
Schmelzpunkt: über 250 ° C
$R_f$-Wert: 0,62 (Reversed Phase Fertigplatte RP8 (E. Merck); Methanol/5%ige Natriumchlorid-Lösung = 6:4)
(7) 4-Amidino-4'-[[(3-carboxy-piperidino)-carbonyl]-methyloxy]-biphenyl
Schmelzpunkt: über 250 ° C
$R_f$-Wert: 0,55 (Reversed Phase Fertigplatte RP8 (E. Merck); Methanol/5%ige Natriumchlorid-Lösung = 6:4)
(8) 4-Amidino-4'-[N-(4-carboxy-butyryl)-N-methyl-amino]-biphenyl
(9) 4-Amidino-4'-[N-(3-carboxy-propionyl)-N-methyl-amino]-biphenyl
(10) 4-Amidino-4'-[(2-carboxy-ethyl)-aminocarbonyl]-biphenyl
(11) 4-Aminomethyl-4'-[N-(4-carboxy-butyryl)-N-methyl-amino]-biphenyl
(12) 3-(5-Carboxy-valerylamino)-3'-guanidino-biphenyl
(13) 4-Amidino-4'-[N-[(carboxymethylamino)-carbonylmethyl]-N-methyl-amino]-biphenyl
(14) 4-Amidino-3'-brom-4'-(4-carboxy-butyrylamino)-biphenyl
(15) 4-Amidino-4'-(4-carboxy-butyrylamino)-3',5'-dibrombiphenyl
(16) 4-Amidino-4'-(5-carboxy-pentyloxy)-3'-methoxy-biphenyl
(17) 4-Amidino-4'-[[N-(3-carboxy-2-propenyl)-N-methyl-amino]-carbonyl]-biphenyl
(18) 4-(5-Carboxy-pentyloxy)-4'-(N-methyl-amidino)-biphenyl
(19) 4-(5-Carboxy-pentyloxy)-4'-(N-methoxy-amidino)-biphenyl

(20) 4-(5-Carboxy-pentyloxy)-4'-hydrazidino-biphenyl
(21) 4-[(3-Carboxy-propyl)-aminocarbonyl]-4'-(N-ethoxycarbonyl-amidino)-biphenyl
(22) 4-(N-Benzyloxy-carbonyl-amidino)-4'-[(3-carboxy-propyl)-aminocarbonyl]-biphenyl
(23) 4-Amidino-4'-[[[N-(2-carboxy-ethyl)-N-methyl-amino]-carbonyl]-methyloxy]-biphenyl
(24) 4-Amidino-4'-[(3-carboxy-propylamino)-sulfonyl]-biphenyl
(25) 4-Amidino-4'-[[N-(3-carboxy-propyl)-N-methyl-amino]-sulfonyl]-biphenyl
(26) 4-Amidino-4'-[(2-carboxy-ethylamino)-carbonylamino]-biphenyl
(27) 4-Amidino-4'-[N-[[N-(2-carboxy-ethyl)-N-methyl-amino]-carbonyl]-N-methyl-amino]-biphenyl
(28) 4-Amidino-4'-[(3-carboxymethyl-phenyl)-aminocarbonyl]-biphenyl
(Zur Verseifung wird Lithiumhydroxid verwendet) Schmelzpunkt: über 260 ° C
(29) 4-Amidino-4'-[[3-(2-carboxy-ethyl)-phenyl]-aminocarbonyl]-biphenyl
(Zur Verseifung wird Lithiumhydroxid verwendet) Schmelzpunkt: über 260 ° C
(30) 4-Amidino-4'-[[3-(2-carboxy-ethyl)-phenyl]-aminocarbonylmethyl]-biphenyl
(Zur Verseifung wird Lithiumhydroxid verwendet) Schmelzpunkt: 262-264 ° C
(31) 4-Amidino-4'-[(4-carboxymethyl-piperidino)-carbonyl]-biphenyl-hydrochlorid
(zur Verseifung wird Lithiumhydroxid verwendet) Schmelzpunkt: 241-245 ° C (Zers.)
(32) 4-Amidino-4'-[(3-carboxymethyl-piperidino)-carbonyl]-biphenyl-hydrochlorid
(zur Verseifung wird Lithiumhydroxid verwendet) Schmelzpunkt: 240-244 ° C (Zers.)
(33) 4-Amidino-4'-[(3-carboxymethyl-pyrrolidino)-carbonyl]-biphenyl
(34) 4-Amidino-4'-[(4-carboxy-piperidino)-carbonylmethyl]-biphenyl-hydrochlorid
(zur Verseifung wird Lithiumhydroxid verwendet) Schmelzpunkt: 260-264 ° C (Zers.)
(35) 4-Amidino-4'-[(3-carboxymethyl-piperidino)-carbonylmethyl]-biphenyl-hydrochlorid
(zur Verseifung wird Lithiumhydroxid verwendet),
$R_f$-Wert: 0,16 (Kieselgel; Methylenchlorid/Ethanol = 4:1)
(36) 4-Amidino-4'-[(3-carboxymethyl-pyrrolidino)-carbonylmethyl]-biphenyl
(37) 4-Amidino-4'-[(3-carboxy-piperidino)-carbonylmethyl]-biphenyl
(38) 4-Amidino-4'-(5-carboxy-pentyloxy)-3'-methansulfonyl-amino-biphenyl
(39) 4-Amidino-3'-benzolsulfonylamino-4'-(5-carboxy-pentyloxy)-biphenyl
(40) 4-Amidino-4'-(4-carboxy-butylthio)-biphenyl
(41) 4-Amidino-4'-[(4-carboxy-butyl)-sulfinyl]-biphenyl
(42) 4-Amidino-4'-[(4-carboxy-butyl)-sulfonyl]-biphenyl
(43) 4-Amidino-4'-(5-carboxy-pentyloxy)-3'-hydroxy-biphenyl
(44) 4-Amidino-4'-(5-carboxy-pentyloxy)-3'-methylthio-biphenyl
(45) 4-Amidino-4'-(5-carboxy-pentyloxy)-3'-methylsulfinyl-biphenyl
(46) 4-Amidino-4'-(5-carboxy-pentyloxy)-3'-methylsulfonyl-biphenyl
(47) 4-Amidino-4'-[(4-carboxymethyl-piperidino)-carbonylmethyl]-biphenyl-hydrochlorid
(zur Verseifung wird Lithiumhydroxid verwendet)
(48) 4-Amidino-4'-[(4-carboxy-butyl)-aminocarbonyl]-biphenyl-hydrochlorid
(zur Verseifung wird Lithiumhydroxid verwendet)
Schmelzpunkt: 275-278 ° C (Zers.)
(49) 4-Amidino-4'-(4-carboxy-butyloxy)-biphenyl
Schmelzpunkt: über 300 ° C

| | | | | | | |
|---|---|---|---|---|---|---|
| Ber. (x 0,1 $H_2O$):<br>Gef.: | C | 68,80<br>68,80 | H | 6,45<br>6,47 | N | 8,92<br>8,52 |

(50) 4-Amidino-4'-[(4-carboxymethyl-piperazino)-carbonyl]-biphenyl-hydrochlorid
(zur Verseifung wird Lithiumhydroxid verwendet)
Schmelzpunkt: über 300 ° C

| | | | | | | | | |
|---|---|---|---|---|---|---|---|---|
| Ber. x 1 HCl:<br>Gef.: | C | 59,62<br>59,09 | H | 5,75<br>5,80 | Cl | 8,80<br>9,15 | N | 13,91<br>13,30 |

(51) 4-Amidino-4'-[[4-(2-carboxy-ethyl)-piperazino]-carbonyl]-biphenyl-dihydrochlorid
(zur Verseifung wird Lithiumhydroxid verwendet)
Schmelzpunkt: 282-286 ° C (Zers.)

(52) 4-Amidino-4'-[(2-carboxy-ethyl)-aminosulfonylamino]-biphenyl
(zur Verseifung wird Lithiumhydroxid verwendet)
Schmelzpunkt: über 265 °C
$R_f$-Wert: 0,84 (Reversed Phase Fertigplatte RP8 (E. Merck); Methanol/10%ige Natriumchloridlösung = 60:40)
(53) 4-Butylamidino-4'-[(4-carboxymethyl-piperidino)-carbonyl]-biphenyl
(54) 4-[(4-Carboxymethyl-piperidino)-carbonyl]-4'-methyl-amidino-biphenyl
(55) 4-Amidino-4'-[(4-carboxymethylen-piperidino)-carbonyl]-biphenyl
Schmelzpunkt: 317-319 °C (Zers.)
(56) 4-Amidino-4'-(4-carboxymethyloxy-phenyl)-biphenyl
(57) 4-Amidino-4'-[(4-carboxy-piperidino)-carbonyl]-biphenyl-hydrochlorid
(zur Verseifung wird Lithiumhydroxid verwendet)
Schmelzpunkt: 303-308 °C (Zers.)
(58) 4-Aminomethyl-4'-[[(4-carboxy-piperidino)-carbonyl]-methyl]-biphenyl
(59) 4-Amidino-4'-[(4,4-bis-carboxymethyl-piperidino)-carbonyl]-biphenyl
(60) 4-Aminomethyl-4'-[(4-carboxymethylen-piperidino)-carbonyl]-biphenyl
(61) 4-Aminomethyl-4'-[(4,4-bis-carboxymethyl-piperidino)-carbonyl]-biphenyl
(62) 4-Amidino-4'-[(4-carboxy-cyclohexyl)-aminocarbonyl]-biphenyl-hydrochlorid
Schmelzpunkt: 344-348 °C (Zers.)
(63) 4-Aminomethyl-3'-[(4-carboxy-butyl)-aminosulfonyl]-4'-methoxy-biphenyl
Schmelzpunkt: 268-270 °C (sintert ab 190 °C)
$R_f$-Wert: 0,14 (Kieselgel; Methylenchlorid/Methanol/konzentriertes Ammoniak = 4:1:0,25)
(64) 4-Amidino-3'-[(4-carboxy-butyl)-aminosulfonyl]-4'-methoxy-biphenyl
$R_f$-Wert: 0,13 (Kieselgel; Methylenchlorid/Methanol/konzentriertes Ammoniak = 2:1:0,25)
(65) 4-Aminomethyl-3'-[(4-carboxy-butyl)-aminocarbonyl]-4'-methoxy-biphenyl
(66) 4-Amidino-3'-[(4-carboxy-butyl)-aminocarbonyl]-4'-methoxy-biphenyl
(67) 4-Amidino-4'-(2-carboxy-ethyloxy)-biphenyl
(68) 4-Amidino-4'-(carboxymethyloxy)-biphenyl
(69) 4-Amidino-4'-[(3,3-bis-carboxymethyl-propyl)-aminocarbonyl]-biphenyl
(70) 4-Amidino-4'-[[N-(3,3-bis-carboxymethyl-propyl)-N-methyl]-aminocarbonyl]-biphenyl
(71) 4-Amidino-3'-[(4-carboxy-cyclohexyl)-aminocarbonyl]-4'-methoxy-biphenyl
$R_f$-Wert: 0,40 (Kieselgel; Methylenchlorid/Methanol = 2:1)
(72) 4-Amidino-3'-[(4-carboxy-cyclohexyl)-aminocarbonyl]-4'-hydroxy-biphenyl
(73) 4-Amidino-3'-[N-(4-carboxy-cyclohexyl)-N-methyl-aminocarbonyl]-4'-methoxy-biphenyl
(74) 4-Amidino-3'-[(4-carboxy-cyclohexyl)-aminosulfonyl]-4'-methoxy-biphenyl
(75) 4-Aminomethyl-3'-[(4-carboxy-cyclohexyl)-aminocarbonyl]-biphenyl
(76) 4-Amidino-4'-[N-(4-carboxy-cyclohexyl)-N-methyl-aminocarbonyl]-biphenyl
Schmelzpunkt: 323-326 °C
(77) 4-Amidino-4'-[N-(3-carboxy-propyl)-N-methyl-aminocarbonyl]-biphenyl
Schmelzpunkt: 282-285 °C (Zers.)
(78) 4-Aminomethyl-3'-[N-(4-carboxy-cyclohexyl)-N-methylaminocarbonyl]-4'-hydroxy-biphenyl
(Zur Verseifung wird Lithiumhydroxid verwendet)
Schmelzpunkt: 245-250 °C (Zers.)
$R_f$-Wert: 0,05 (Kieselgel; Methylenchlorid/Methanol/konz. Ammoniak = 2:1:0,5)
(79) 4-Aminomethyl-3'-[(4-carboxy-cyclohexyl)-aminocarbonyl]-4'-methoxy-biphenyl
(80) 4-Amidino-3'-[N-(4-carboxy-cyclohexyl)-N-methyl-aminocarbonyl]-4'-hydroxy-biphenyl
Schmelzpunkt: ab 290 °C (Zers.)
$R_f$-Wert: 0,34 (Kieselgel; Methylenchlorid/Methanol = 8:2)

Beispiel 2

4-Amidino-4'-(5-carboxy-pentyloxy)-biphenyl-hydrochlorid

0,5 g Di-[4-Amidino-4'-(5-methoxycarbonyl-pentyloxy)-biphenyl]-dihydrogencarbonat werden in 15 ml 8n Salzsäure 5 Wochen bei Raumtemperatur und 5 Tage bei 40 °C gerührt. Man verdünnt mit Wasser, filtriert ab und wäscht den Rückstand mit Wasser und Aceton.
Ausbeute: 0,43 g (92 % der Theorie),
Schmelzpunkt: über 250 °C

| | | | | | | | | |
|---|---|---|---|---|---|---|---|---|
| Ber. (x 0,6 HCl):<br>Gef.: | C | 65,53<br>65,30 | H | 6,54<br>6,51 | N | 8,04<br>7,55 | Cl | 6,11<br>6,31 |

Analog werden folgende Verbindungen erhalten:
(1) 4-Amidino-4'-(3-carboxy-propyloxy)-biphenyl
Schmelzpunkt: über 290 °C

| | | | | | | | | |
|---|---|---|---|---|---|---|---|---|
| Ber. (x 0,75 HCl x 0,25 $H_2O$):<br>Gef.: | C | 61,84<br>61,85 | H | 5,88<br>5,90 | N | 8,48<br>8,46 | Cl | 8,05<br>8,27 |

(2) 4-Amidino-4'-(4-carboxy-butyloxy)-biphenyl
(3) 4-Amidino-4'-(5-carboxy-pentyloxy)-3'-nitro-biphenyl
(4) 4-Amidino-3'-amino-4'-(5-carboxy-pentyloxy)-biphenyl
(5) 3-Acetylamino-4'-amidino-4-(5-carboxy-pentyloxy)-biphenyl
(6) 4-Amidino-3'-benzoylamino-4'-(5-carboxy-pentyloxy)-biphenyl
(7) 4-Amidino-4'-(5-carboxy-pentyloxy)-3-chlor-biphenyl
(8) 4-Amidino-4'-(5-carboxy-pentyloxy)-3-fluor-biphenyl
(9) 4-Amidino-4'-(5-carboxy-pentyloxy)-2',3'-dimethyl-biphenyl
(10) 4-Amidino-4'-(5-carboxy-pentyloxy)-3'-trifluormethyl-biphenyl
(11) 4-Amidino-4'-[[2-carboxy-1-[[2-(4-methoxy-phenyl)-ethyl]-aminocarbonyl]-ethylamino]-carbonylmethyl]-biphenyl-hydrochlorid
(Man arbeitet in einem 1:1-Gemisch aus 2n Salzsäure und Tetrahydrofuran, Reaktionsdauer 72 Stunden)
$R_f$-Wert: 0,50 (Reversed Phase Fertigplatte RP18 (E. Merck); Acetonitril/Wasser/Essigsäure = 5:5:0,1)

Beispiel 3

4-Amidino-4'-[[[2-carboxy-1-[[2-(4-methoxy-phenyl)-ethyl]-aminocarbonyl]-ethylamino]-carbonylmethyl]-aminocarbonyl]-biphenyl-hydrochlorid

0,67 g 4-(N-Benzyloxycarbonyl-amidino)-4'-[[[2-benzyloxycarbonyl-1-[[2-(4-methoxy-phenyl)-ethyl]-aminocarbonyl]-ethylamino]-carbonylmethyl]-aminocarbonyl]-biphenyl werden in 100 ml Methanol mit Wasserstoff von 5 bar Druck in Gegenwart von 0,2 g 5%iger Palladiumkohle bei Raumtemperatur 2 Stunden hydriert. Man filtriert vom Katalysator ab, wäscht mit Methanol und wenig 1n Salzsäure nach und engt das Filtrat ein. Der Rückstand wird mit Ether verrieben und abfiltriert.
Ausbeute: 0,48 g (92 % der Theorie),
Schmelzpunkt: 202-205 °C (Zers.)
Analog werden folgende Verbindungen erhalten:
(1) 4-[(3-Carboxy-propylamino)-carbonyl]-4'-(N-methoxycarbonyl-amidino)-biphenyl
(Als Lösungsmittel dient Dimethylformamid und als Katalysator 10%ige Palladiumkohle)
(2) 4-Amidino-4'-[(4-carboxymethyl-4-hydroxy-piperidino)-carbonyl]-biphenyl
(3) 4-[(4-Carboxymethyl-piperidino)-carbonyl]-4'-(N-methoxycarbonyl-amidino)-biphenyl
Man arbeitet mit 10%iger Palladiumkohle in Dioxan
Schmelzpunkt: 194-196 °C (Zers.)
$R_f$-Wert: 0,14 (Kieselgel; Methylenchlorid/Ethanol = 9:1)

Beispiel 4

4-Amidino-4'-[(4-benzyloxycarbonylmethyl-piperidino)-carbonyl]-biphenyl-toluolsulfonat

Eine Mischung aus 1,6 g 4-Amidino-4'-[(4-carbonylmethyl-piperidino)-carbonyl]-biphenyl-hydrochlorid, 50 ml Benzylalkohol und 1 g p-Toluolsulfonsäure wird bei 100 mbar unter Rühren 4 Stunden auf 70 °C erhitzt und anschließend im Wasserstrahlvakuum bei 140-150 °C eingeengt. Der Rückstand wurde mit Ether verrieben, das erhaltene Festprodukt abfiltriert, in Dimethylformamid gelöst, die Lösung eingeengt und der Rückstand mit Ether verrieben.
Ausbeute: 2,46 g (98 % der Theorie),
Schmelzpunkt: 218-223 °C (Zers.)

Analog werden folgende Verbindungen erhalten, wobei man jeweils in Dimethylformamid mit einem 12-fachen molaren Überschuß des betreffenden Alkohols und einem 15-fachen Überschuß p-Toluolsulfonsäure arbeitet:

(1) 4-Amidino-4'-[[4-(n-butyloxycarbonylmethyl)-piperidino]-carbonyl]-biphenyl-toluolsulfonat
(2) 4-Amidino-4'-[[4-(2-phenyl-ethyl)-oxycarbonylmethyl-piperidino]-carbonyl]-biphenyl-toluolsulfonat
(3) 4-Amidino-4'-[[4-[2-(2-oxo-pyrrolidinyl)-ethyl]-oxycarbonylmethyl-piperidino]-carbonyl]-biphenyl-toluolsulfonat
(4) 4-Amidino-4'-[[4-[(3-pyridyl)-methyl]-oxycarbonylmethyl-piperidino]-carbonyl]-biphenyl-toluolsulfonat
(5) 4-Amidino-4'-[[4-(2-morpholino-ethyl)-oxycarbonylmethyl-piperidino]-carbonyl]-biphenyl-toluolsulfonat
(6) 4-Amidino-4'-[[4-(2-thiomorpholino-ethyl)-oxycarbonylmethyl-piperidino]-carbonyl]-biphenyl
(7) 4-[[4-(n-Butyloxycarbonyl-methyl)-piperidino]-carbonyl]-4'-(N-methyl-amidino)-biphenyl

Beispiel 5

Di-[4-Amidino-4'-(5-methoxycarbonyl-pentyloxy)-biphenyl]-dihydrogencarbonat

Man überschichtet 75 ml Methanol mit 30 ml Petroläther und leitet unter Eiskühlung Chlorwasserstoffgas bis zur Sättigung ein. Man trägt nun 2,1 g 4-Cyano-4'-(5-ethoxycarbonylpentyloxy)-biphenyl ein und rührt 18 Stunden bei Raumtemperatur. Man engt im Vakuum zur Trockene ein, suspendiert den Rückstand in Methanol, setzt 5,36 g Ammoniumcarbonat zu und rührt 16 Stunden bei Raumtemperatur. Der erhaltene Niederschlag wird abfiltriert und durch Verrühren mit Methylenchlorid/Methanol (85:15) und Wasser gereinigt.
Ausbeute: 1,75 g (75 % der Theorie),
Schmelzpunkt: 185-189 ° C (Zers.)

| | | | | | | |
|---|---|---|---|---|---|---|
| Ber. (x 0,5 $H_2CO_3$):<br>Gef.: | C | 66,31<br>66,75 | H | 6,74<br>6,85 | N | 7,55<br>7,41 |

Analog werden folgende Verbindungen erhalten:

(1) 4-Amidino-4'-(4-methoxycarbonyl-butyrylamino)-biphenyl-hydrochlorid
Schmelzpunkt: ab 210 ° C (Zers.)
$R_f$-Wert: 0,13 (Kieselgel; Essigester/Ethanol = 7:3)
(2) 4-Amidino-4'-[[(4-methoxycarbonyl-methyl)-aminocarbonyl]-methyloxy]-biphenyl-hydrochlorid
Schmelzpunkt: 223-225 ° C (Zers.)
$R_f$-Wert: 0,06 (Kieselgel; Methylenchlorid/Methanol = 10:1)
(3) 4-Amidino-4'-[[(2-methoxycarbonyl-ethyl)-aminocarbonyl]-methyloxy]-biphenyl-hydrochlorid
Schmelzpunkt: 155 ° C (Zers.)
$R_f$-Wert: 0,18 (Kieselgel; Methylenchlorid/Methanol = 10:1)
(4) 4-Amidino-4'-[[3-(methoxycarbonyl-methyl)-phenyl]-aminocarbonyl]-biphenyl-hydrochlorid
Schmelzpunkt: über 260 ° C
$R_f$-Wert: 0,27 (Kieselgel; Methylenchlorid/Ethanol = 4:1)
(5) 4-Amidino-4'-[(2-methoxycarbonyl-ethyl)-aminocarbonyl-methyl]-biphenyl
[Das Produkt wird durch Chromatographie an Kieselgel (Elutionsmittel: Methylenchlorid/Ethanol/konzentriertes Ammoniak = 4:1:0,25) gereinigt]
$R_f$-Wert: 0,20 (Kieselgel; Methylenchlorid/Ethanol/konzentriertes Ammoniak = 4:1:0,25)
(6) 4-Amidino-4'-[(3-methoxycarbonyl-propyl)-aminocarbonyl]-biphenyl-hydrochlorid
Schmelzpunkt: 210-212 ° C
$R_f$-Wert: 0,17 (Kieselgel; Methylenchlorid/Ethanol/konzentriertes Ammoniak = 4:1:0,25)
(7) 4-Amidino-4'-[N-(3-methoxycarbonyl-butyryl)-N-methylamino]-biphenyl-hydrochlorid
(8) 4-Amidino-4'-(3-methoxycarbonyl-propyloxy)-biphenyl-dihydrogencarbonat
Schmelzpunkt: 203-205 ° C
(9) 4-Amidino-4'-(4-methoxycarbonyl-butyloxy)-biphenyl-hydrochlorid
(Zur Umwandlung des intermediär gebildeten Iminoesters in das Amidin verwendet man ein 10:1-Gemisch aus Methanol und konzentriertem wäßrigen Ammoniak)
Schmelzpunkt: 190-194 ° C
(10) 4-Amidino-4'-[N-(3-methoxycarbonyl-propionyl)-N-methylamino]-biphenyl-hydrochlorid
(11) 4-Amidino-4'-[(2-methoxycarbonyl-ethyl)-aminocarbonyl]-biphenyl-hydrochlorid

(12) 4-Amidino-4'-[N-[(methoxycarbonyl-methyl)-aminocarbonylmethyl]-N-methyl-amino]-biphenyl-hydrochlorid
(13) 4-Amidino-3'-brom-4'-[(4-methoxycarbonyl-butyryl)-amino]-biphenyl-hydrochlorid
(14) 4-Amidino-3',5'-dibrom-4'-[(4-methoxycarbonyl-butyryl)-amino]-biphenyl-hydrochlorid
(15) 4-Amidino-4'-(5-methoxycarbonyl-pentyloxy)-3'-nitro-biphenyl-hydrochlorid
(16) 4-Amidino-3'-amino-4'-(5-methoxycarbonyl-pentyloxy)-biphenyl-hydrochlorid
(17) 3-Acetylamino-4'-amidino-4-(5-methoxycarbonyl-pentyloxy)-biphenyl-hydrochlorid
(18) 4-Amidino-3'-benzoylamino-4'-(5-methoxycarbonyl-pentyloxy)-biphenyl-hydrochlorid
(19) 4-Amidino-3'-methansulfonylamino-4'-(5-methoxycarbonyl-pentyloxy)-biphenyl-hydrochlorid
(20) 4-Amidino-3'-benzolsulfonylamino-4'-[(5-methoxycarbonyl-pentyloxy)-biphenyl-hydrochlorid
(21) 4-Amidino-4'-(4-methoxycarbonyl-butylthio)-biphenyl-hydrochlorid
(22) 4-Amidino-4'-[(4-methoxycarbonyl-butyl)-sulfonyl]-biphenyl-hydrochlorid
(23) 4-Amidino-3'-hydroxy-4'-(5-methoxycarbonyl-pentyloxy)-biphenyl-hydrochlorid
(24) 4-Amidino-3'-methoxy-4'-(5-methoxycarbonyl-pentyloxy]-biphenyl-hydrochlorid
(25) 4-Amidino-3-chlor-4'-(5-methoxycarbonyl-pentyloxy)-biphenyl-hydrochlorid
(26) 4-Amidino-3-fluor-4'-(5-methoxycarbonyl-pentyloxy)-biphenyl-hydrochlorid
(27) 4-Amidino-2',3'-dimethyl-4'-(5-methoxycarbonyl-pentyloxy)-biphenyl-hydrochlorid
(28) 4-Amidino-4'-(5-methoxycarbonyl-pentyloxy)-3'-trifluormethyl-biphenyl-hydrochlorid
(29) 4-Amidino-4'-[[N-(3-methoxycarbonyl-2-propenyl)-N-methyl-amino]-carbonyl]-biphenyl-hydrochlorid
(30) 4-[(5-Methoxycarbonyl-pentyloxy)-4'-(N-methyl-amidino)-biphenyl
(Der Iminoester wird in absolutem Methanol aufgenommen und mit einem 20-fachen Überschuß einer methanolischen Methylaminlösung umgesetzt)
(31) 4-Amidino-4'-[[[N-(2-methoxycarbonyl-ethyl)-N-methylamino]-carbonyl]-methyloxy]-biphenyl-hydrochlorid
(32) 4-Amidino-4'-[(3-methoxycarbonyl-propylamino)-sulfonyl]-biphenyl-hydrochlorid
(33) 4-Amidino-4'-[[N-(3-methoxycarbonyl-propyl)-N-methylamino]-sulfonyl]-biphenyl-hydrochlorid
(34) 4-Amidino-4'-[(2-methoxycarbonyl-ethyl)-aminocarbonyl-amino]-biphenyl-hydrochlorid
(35) 4-Amidino-4'-[N-[[N-(2-methoxycarbonyl-ethyl)-N-methylamino]-carbonyl]-N-methyl-amino]-biphenyl-hydrochlorid
(36) 4-Amidino-4'-[[3-(2-methoxycarbonyl-ethyl)-phenyl]-aminocarbonyl]-biphenyl-hydrochlorid
Schmelzpunkt: 266-268 °C (Zers.)
(37) 4-Amidino-4'-[[[3-(methoxycarbonyl-methyl)-phenyl]-aminocarbonyl]-methyl]-biphenyl-hydrochlorid
$R_f$-Wert: 0,48 (Kieselgel; Methylenchlorid/Ethanol = 4:1)
(38) 4-Amidino-4'-[[[3-(2-methoxycarbonyl-ethyl)-phenyl]-aminocarbonyl]-methyl]-biphenyl-hydrochlorid
$R_f$-Wert: 0,38 (Kieselgel; Methylenchlorid/Ethanol = 4:1)
(39) 4-Amidino-4'-[[(4-methoxycarbonyl-piperidino)-carbonyl]-methyloxy]-biphenyl-hydrochlorid
Schmelzpunkt: 240 °C (Zers.)
$R_f$-Wert: 0,33 (Kieselgel; Methylenchlorid/Ethanol = 9:1)
(40) 4-Amidino-4'-[[(3-methoxycarbonyl-piperidino)-carbonyl]-methyloxy]-biphenyl-hydrochlorid
$R_f$-Wert: 0,78 (Kieselgel; Methylenchlorid/Ethanol = 8:2)
(41) 4-Amidino-4'-[(4-methoxycarbonylmethyl-piperidino)-carbonyl]-biphenyl-hydrochlorid
Schmelzpunkt: 268-270 °C
(42) 4-Amidino-4'-[(3-methoxycarbonylmethyl-piperidino)-carbonyl]-biphenyl-hydrochlorid
Schmelzpunkt: 277-280 °C
(43) 4-Amidino-4'-[(3-methoxycarbonylmethyl-pyrrolidino)-carbonyl]-biphenyl-hydrochlorid
(44) 4-Amidino-4'-[(4-methoxycarbonyl-piperidino)-carbonylmethyl]-biphenyl-hydrochlorid
Schmelzpunkt: 224-228 °C (Zers.)
(45) 4-Amidino-4'-[(3-methoxycarbonylmethyl-piperidino)-carbonylmethyl]-biphenyl-hydrochlorid
Schmelzpunkt: 166-172 °C
(46) 4-Amidino-4'-[(3-methoxycarbonylmethyl-pyrrolidino)-carbonylmethyl]-biphenyl-hydrochlorid
(47) 4-Amidino-4'-[(3-methoxycarbonyl-piperidino)-carbonylmethyl]-biphenyl-hydrochlorid
(48) 4-Amidino-4'-(4-sulfo-butyloxy)-biphenyl
(49) 4-Amidino-4'-(4-phosphono-butyloxy)-biphenyl-hydrochlorid
(50) 4-Amidino-4'-[4-(O-methyl-phosphono)-butyloxy]-biphenyl-hydrochlorid
(51) 4-Amidino-4'-(5-methoxycarbonyl-pentyloxy)-3'-methylthio-biphenyl-hydrochlorid
(52) 4-Amidino-4'-(5-methoxycarbonyl-pentyloxy)-3'-methylsulfonyl-biphenyl-hydrochlorid
(53) 4-Amidino-4'-[3-(5-tetrazolyl)-propyloxy]-biphenyl-hydrochlorid

(54) 4-Amidino-4'-[[2-methoxycarbonyl-1-[[2-(4-methoxyphenyl)-ethyl]-aminocarbonyl]-ethylamino]-carbonylmethyl]-biphenyl
$R_f$-Wert: 0,11 (Kieselgel; Methylenchlorid/Methanol = 9:1)
(55) 4-Amidino-4'-[(4-methoxycarbonylmethyl-piperidino)-carbonylmethyl]-biphenyl-hydrochlorid
Schmelzpunkt: 172-177 °C
(56) 4-Amidino-4'-[(4-methoxycarbonylbutyl)-aminocarbonyl]-biphenyl-hydrochlorid
Schmelzpunkt: 208-212 °C
(57) 4-Amidino-4'-[(4-ethoxycarbonylmethyl-piperidino)-carbonyl]-biphenyl
$R_f$-Wert: 0,19 (Kieselgel; Methylenchlorid/Ethanol = 4:1)
(58) 4-Amidino-4'-[(4-methoxycarbonylmethyl-piperazino)-carbonyl]-biphenyl-dihydrochlorid
Schmelzpunkt: 274-276 °C
(59) 4-Amidino-4'-[[(4-(2-methoxycarbonyl-ethyl)-piperazino]-carbonyl]-biphenyl-dihydrochlorid
Schmelzpunkt: 292-296 °C (Zers.)

| | | | | | | | | |
|---|---|---|---|---|---|---|---|---|
| Ber. (x 2 HCl x $H_2O$):<br>Gef.: | C | 54,43<br>54,44 | H | 6,23<br>5,93 | N | 11,54<br>11,50 | Cl | 14,61<br>14,41 |

(60) 4-Amidino-4'-[(2-methoxycarbonyl-ethyl)-aminosulfonylamino]-biphenyl-hydrochlorid
Schmelzpunkt: sintert ab 176 °C (Zers.)

| | | | | | | | | | | |
|---|---|---|---|---|---|---|---|---|---|---|
| Ber.:<br>Gef.: | C | 48,63<br>48,58 | H | 5,32<br>5,27 | N | 12,97<br>12,66 | S | 7,42<br>7,51 | Cl | 9,03<br>8,81 |

(61) 4-(N-Butyl-amidino)-4'-[(4-methoxycarbonylmethyl-piperidino)-carbonyl]-biphenyl
(In der zweiten Stufe der Reaktion wird n-Butylamin eingesetzt).
(62) 4-[(4-Methoxycarbonylmethyl-piperidino)-carbonyl]-4'-(N-methyl-amidino)-biphenyl
(In der zweiten Stufe der Reaktion wird wäßrige Methylaminlösung eingesetzt).
(63) 4-Amidino-4'-[(4-methoxycarbonylmethylen-piperidino)-carbonyl]-biphenyl-hydrochlorid
Schmelzpunkt: 298-300 °C (Zers.)
(64) 4-Amidino-4'-(4-methoxycarbonylmethyloxy-phenyl)-biphenyl
(65) 4-Amidino-4'-[(4-methoxycarbonyl-piperidino)-carbonyl]-biphenyl
Schmelzpunkt: 294-296 °C (Zers.)
(66) 4-Amidino-4'-[(4-dimethylaminocarbonylmethyl-piperidino)-carbonyl]-biphenyl
(67) 4-Amidino-4'-[(4-sulfomethyl-piperidino)-carbonyl]-biphenyl
Schmelzpunkt:
(68) 4-Amidino-4'-[(4-(5-tetrazolyl-methyl)-piperidino]-carbonyl]-biphenyl
(69) 4-Amidino-4'-[(4,4-bis-methoxycarbonylmethyl-piperidino)-carbonyl]-biphenyl
(70) 4-Amidino-4'-(aminocarbonylmethyl-aminocarbonyl)-biphenyl-hydrochlorid (Hergestellt aus 4-Cyano-4'-(methoxycarbonylmethyl-aminocarbonyl)-biphenyl)
Schmelzpunkt: über 260 °C
$R_f$-Wert: 0,28 (Kieselgel; Methylenchlorid/Ethanol = 4:1)
(71) 4-Amidino-4'-[(4-methoxycarbonyl-cyclohexyl)-aminocarbonyl]-biphenyl-hydrochlorid
Schmelzpunkt: 302-305 °C (Zers.)
(72) 4-Amidino-4'-(2-methoxycarbonyl-ethyloxy)-biphenyl
(73) 4-Amidino-4'-(methoxycarbonyl-methyloxy)-biphenyl
(74) 4-Amidino-4'-[(3,3-bis-methoxycarbonylmethyl-propyl)-aminocarbonyl]-biphenyl
(75) 4-Amidino-4'-[N-(3,3-bis-methoxycarbonylmethyl-propyl)-N-methyl-aminocarbonyl]-biphenyl
(76) 4-Amidino-4'-methoxy-3'-[(4-methoxycarbonyl-butyl)-aminosulfonyl]-biphenyl-hydrochlorid
$R_f$-Wert: 0,19 (Kieselgel; Methylenchlorid/Methanol/konzentriertes Ammoniak = 4:1:0,25)
(77) 4-Amidino-4'-methoxy-3'-[(4-methoxycarbonyl-butyl)-aminocarbonyl]-biphenyl
(78) 4-Amidino-4'-methoxy-3'-[(4-methoxycarbonyl-cyclohexyl)-aminocarbonyl]-biphenyl
$R_f$-Wert: 0,15 (Kieselgel; Methylenchlorid/Methanol = 9:1)
(79) 4-Amidino-4'-hydroxy-3'-[(4-methoxycarbonyl-cyclohexyl)-aminocarbonyl]-biphenyl
(80) 4-Amidino-4'-methoxy-3'-[N-(4-methoxycarbonyl-cyclohexyl)-N-methyl-aminocarbonyl]-biphenyl
(81) 4-Amidino-4'-methoxy-3'-[(4-methoxycarbonyl-cyclohexyl)-aminosulfonyl]-biphenyl

(82) 4-Amidino-4'-[N-(4-methoxycarbonyl-cyclohexyl)-N-methylaminocarbonyl]-biphenyl-hydrochlorid
Schmelzpunkt: 295-300 °C
(83) 4-Amidino-4'-[N-(3-methoxycarbonyl-propyl)-N-methylaminocarbonyl]-biphenyl
Schmelzpunkt: 235-238 °C (Zers.)
(84) 4-Amidino-4'-hydroxy-3'-[N-(4-methoxycarbonyl-cyclohexyl)-N-methyl-aminocarbonyl]-biphenyl
Schmelzpunkt: ab 195 °C (Zers.)
$R_f$-Wert: 0,12 (Kieselgel; Methylenchlorid/Methanol = 9:1)

Beispiel 6

4-Hydrazidino-4'-(5-methoxycarbonyl-pentyloxy)-biphenyl

Man setzt 4-Amidino-4'-(5-methoxycarbonyl-pentyloxy)-biphenyl-hydrochlorid mit einem 30-fachen Überschuß an Hydrazin in Methanol bei Raumtemperatur um. Die Reaktionsdauer beträgt 3 Tage.

Analog wird folgende Verbindung erhalten:
(1) 4-(N-Methoxy-amidino)-4'-(5-methoxycarbonyl-pentyloxy)-biphenyl
(Man verwendet O-Methyl-hydroxylamin-hydrochlorid und Ethyldiisopropylamin als Base)

Beispiel 7

4-(N-Ethoxycarbonyl-amidino)-4'-[(4-methoxycarbonylmethyl-piperidino)-carbonyl]-biphenyl

0,4 g 4-Amidino-4'-[(4-methoxycarbonylmethyl-piperidino)-carbonyl]-biphenyl-hydrochlorid und 0,11 ml Chlorameisensäureethylester in 80 ml Methylenchlorid werden unter starkem Rühren so mit 0,1 N Natronlauge versetzt, daß der pH-Wert der Mischung auf 9 gehalten wird. Die organische Phase wird abgetrennt und zur Trockne eingedampft.
Ausbeute: 0,27 g (60 % der Theorie),
$R_f$-Wert: 0,60 (Kieselgel; Methylenchlorid/Ethanol = 9:1)
Analog werden folgende Verbindungen erhalten:
(1) 4-(N-Methoxycarbonyl-amidino)-4'-[(3-methoxycarbonyl-propylamino)-carbonyl]-biphenyl
(2) 4-[(3-Benzyloxycarbonyl-propyl)-aminocarbonyl]-4'-(N-methoxycarbonyl-amidino)-biphenyl
(3) 4-(N-Benzyloxycarbonyl-amidino)-4'-[(3-methoxycarbonyl-propyl)-aminocarbonyl]-biphenyl
(4) 4-(N-Ethoxycarbonyl-amidino)-4'-[(3-methoxycarbonyl-propyl)-aminocarbonyl]-biphenyl
(5) 4-(N-Methoxycarbonyl-amidino)-4'-[(4-methoxycarbonyl-methyl-piperidino)-carbonyl]-biphenyl
$R_f$-Wert: 0,59 (Kieselgel; Methylenchlorid/Ethanol = 9:1)
(6) 4-(N-Benzyloxycarbonyl-amidino)-4'-[(4-methoxycarbonyl-methyl-piperidino)-carbonyl]-biphenyl
Schmelzpunkt: 139-142 °C,
$R_f$-Wert: 0,66 (Kieselgel; Methylenchlorid/Methanol = 9:1)
(7) 4-(N-Ethoxycarbonyl-amidino)-4'-[(4-ethoxycarbonyl-methyl-piperidino)-carbonyl]-biphenyl
Schmelzpunkt: 126-130 °C,
$R_f$-Wert: 0,57 (Kieselgel; Methylenchlorid/Ethanol = 9:1)
(8) 4-(N-Benzyloxycarbonylmethyl-piperidino)-carbonyl]-4'-(N-methoxycarbonyl-amidino)-biphenyl
Schmelzpunkt: 126-128 °C,
$R_f$-Wert: 0,63 (Kieselgel; Cyclohexan/Essigester = 1:3)
(9) 4-(N-Methoxycarbonyl-amidino)-4'-[(4-methoxycarbonyl-cyclohexyl)-aminocarbonyl]-biphenyl
Schmelzpunkt: 349-351 °C (Zers.)
(10) 4-(N-Methoxycarbonyl-amidino)-4'-[N-(4-methoxycarbonyl-cyclohexyl)-N-methyl-aminocarbonyl]-biphenyl
Schmelzpunkt: 218-220 °C

Beispiel 8

4-Amidino-4'-[(4-methoxycarbonyl-butyl)-sulfinyl]-biphenyl

Hergestellt aus 4-Amidino-4'-(4-methoxycarbonyl-butylthio)-biphenyl durch Oxidation mit m-Chlor-perbenzoesäure in Methylenchlorid bei -20 °C während 15 Stunden.

Analog werden folgende Verbindungen erhalten:
(1) 4-Amidino-4'-[[4-[[2-(1-oxido-thiomorpholino)-ethyl]-oxy-carbonylmethyl]-piperidino]-carbonyl]-biphenyl

(2) 4-Amidino-4'-(5-methoxycarbonyl-pentyloxy)-3'-methyl-sulfinyl-biphenyl

Beispiel 9

4-Aminomethyl-4'-[(4-methoxycarbonylmethyl-piperidino)-carbonyl]-biphenyl-hydrochlorid

1,89 g 4-Cyano-4'-[(4-methoxycarbonylmethyl-piperidino)-carbonyl]-biphenyl werden in 40 ml Methanol gelöst, dem 2 ml methanolische Salzsäure zugesetzt waren. Man hydriert mit Wasserstoff von 5 bar Druck bei Raumtemperatur in Gegenwart von 0,4 g 10%iger Palladiumkohle. Nach 2,2 Stunden filtriert man vom Katalysator ab und engt ein. Der Rückstand wird durch Chromatographie an Kieselgel gereinigt (Elutionsmittel; Methylenchlorid/Methanol = 9:1)
carbonyl]-biphenyl
Schmelzpunkt: 129-132 °C

(2) 4-Cyano-4'-[[[3-(methoxycarbonyl-methyl)-phenyl]-aminocarbonyl]-methyl]-biphenyl
Schmelzpunkt: 142-144 °C
(3) 4-Cyano-4'-[[3-(methoxycarbonyl-methyl)-phenyl]-aminocarbonyl]-biphenyl
(als Lösungsmittel wird Dimethylformamid verwendet)
Schmelzpunkt: 148-149 °C
(4) 4-Cyano-4'-[(methoxycarbonyl-methyloxy)-methylcarbonyl-amino]-biphenyl
(5) 4-Cyano-4'-[[N-(methoxycarbonyl-methyl)-N-methyl-amino]-methylcarbonylamino]-biphenyl
(6) 4-Cyano-4'-[(4-methoxycarbonylmethyl-piperidino)-carbonyl]-biphenyl
Schmelzpunkt: 130-134 °C
(7) 4-Cyano-4'-[(3-methoxycarbonylmethyl-piperidino)-carbonyl]-biphenyl
Schmelzpunkt: 130-134 °C
(8) 4-Cyano-4'-[(3-methoxycarbonylmethyl-piperidino)-carbonylmethyl]-biphenyl
$R_f$-Wert: 0,71 (Kieselgel; Methylenchlorid/Ethanol = 9:1)
(9) 4-Cyano-4'-[(4-methoxycarbonylmethyl-piperidino)-carbonylmethyl]-biphenyl
Schmelzpunkt: 116-118 °C
(10) 4-Cyano-4'-[(4-methoxycarbonyl-butyl)-aminocarbonyl]-biphenyl
Schmelzpunkt: 148-150 °C
(11) 4-Cyano-4'-[[4-(2-methoxycarbonyl-ethyl)-piperazino]-carbonyl]-biphenyl
$R_f$-Wert: 0,62 (Kieselgel; Methylenchlorid/Ethanol = 9:1)
(12) 4-Cyano-4'-[(4-methoxycarbonylmethyl-piperazino)-carbonyl]-biphenyl
Schmelzpunkt: 170-172 °C
(13) 4-Cyano-4'-[(4-methoxycarbonyl-cyclohexyl)-aminocarbonyl]-biphenyl
Schmelzpunkt: 273-275 °C (Zers.)
(14) 4-(N-Benzyloxycarbonyl-amidino)-4'-[[[2-benzyloxycarbonyl-1-[[2-(4-methoxy-phenyl)-ethyl]-aminocarbonyl]-ethylamino]-carbonylmethyl]-aminocarbonyl]-biphenyl
Schmelzpunkt: 162-165 °C

Zur Herstellung der 4'-(N-Benzyloxycarbonyl-amidino)-biphenyl-4-carbonsäure geht man von 4'-Cyano-biphenyl-4-carbonsäure-methylester aus, der analog Beispiel 6 in den 4'-Amidino-biphenyl-4-carbonsäuremethylester übergeführt wird. Dieser wird in das N-Benzyloxycarbonyl-derivat überführt, indem man ihn in einem 4:1-Gemisch aus Methylenchlorid und Methanol in Gegenwart von 1N Natronlauge mit Chlorameisensäurebenzylester umsetzt. Die Verseifung des Methylesters zur Carbonsäure geschieht mit Lithiumhydroxid.

Glycyl-asparaginsäure-$\beta$-benzylester-$\alpha$-[2-(4-methoxyphenyl)-ethyl]-amid wird nach bekannten Verfahren der Peptidsynthese hergestellt, indem man zunächst nach Beispiel 15 N-tert-Butyloxycarbonyl-asparaginsäure-$\beta$-benzylester mit 2-(4-Methoxyphenyl)-ethylamin kondensiert, das Produkt nach Entschützung der Aminofunktion des Asparaginsäureteils mit N-tert-Butyloxycarbonyl-glycin wiederum analog Beispiel 15 umsetzt und wieder entschützt.

(15) 4-Cyano-4'-hydroxy-3'-[N-(4-methoxycarbonyl-cyclohexyl)-N-methyl-aminocarbonyl]-biphenyl
$R_f$-Wert: 0,40 (Kieselgel; Cyclohexan/Essigester = 7:3)
(16) 4-Cyano-4'-[N-(4-methoxycarbonyl-cyclohexyl)-N-methylaminocarbonyl]-biphenyl
Schmelzpunkt: 218-220 °C

Beispiel 12

4-Cyano-4'-(5-ethoxycarbonyl-pentyloxy)-biphenyl

1,95 g 4-Cyano-4'-hydroxy-biphenyl werden in 25 ml Dimethylformamid gelöst. Zu der auf 0°C abgekühlten Mischung gibt man 0,44 g einer 55%igen Dispersion von Natriumhydrid in Öl und rührt 0,5 Stunden. Man fügt 1,28 g 6-Brom-capronsäure und weitere 10 ml Dimethylformamid zu und rührt 2 Stunden bei Raumtemperatur. Das Dimethylformamid wird im Vakuum abdestilliert, der Rückstand mit Wasser verrieben, der gebildete Niederschlag abfiltriert und aus Ethanol umkristallisiert.
Ausbeute: 2,1 g (62 % der Theorie),
Schmelzpunkt: sintert ab 62°C

| | | | | | | |
|---|---|---|---|---|---|---|
| Ber.: | C | 74,75 | H | 6,87 | N | 4,15 |
| Gef.: | | 74,40 | | 6,76 | | 4,24 |

Analog werden folgende Verbindungen erhalten:

(1) 4-Cyano-4'-(3-methoxycarbonyl-propyloxy)-biphenyl Schmelzpunkt: 107-108°C
(2) 4-Cyano-4'-(4-methoxycarbonyl-butyloxy)-biphenyl Schmelzpunkt: 97-99°C
(3) 4-Cyano-4'-(5-ethoxycarbonyl-pentyloxy)-3'-nitro-biphenyl
(4) 4-Cyano-4'-(4-methoxycarbonyl-butylthio)-biphenyl
(5) 3-Chlor-4-cyano-4'-(5-ethoxycarbonyl-pentyloxy)-biphenyl
(6) 4-Cyano-4'-(5-ethoxycarbonyl-pentyloxy)-3-fluor-biphenyl
(7) 4-Cyano-2',3'-dimethyl-4'-(5-ethoxycarbonyl-pentyloxy)-biphenyl
(8) 4-Cyano-4'-(5-ethoxycarbonyl-pentyloxy)-3'-trifluor-methyl-biphenyl
(9) 4-Cyano-4'-[[(2-methoxycarbonyl-ethyl)-aminocarbonyl]-methyloxy]-biphenyl
(4-Cyano-4'-[[[N-(2-methoxycarbonyl-ethyl)-N-methyl-amino]-carbonyl]-methyloxy]-biphenyl erhält man aus 4-Cyano-4'-[[(2-methoxycarbonyl-ethyl)-aminocarbonyl]-methyloxy]-biphenyl durch Methylierung mit Methyljodid)
(10) 4-[2-[N-Acetyl-N-(2-methoxycarbonyl-ethylamino)-ethyloxy]-4'-cyano-biphenyl
(11) 4-Cyano-4'-[2-[N-(2-methoxycarbonyl-ethyl)-N-methansulfonyl-amino]-ethyloxy]-biphenyl
(12) 4-[2-[N-Benzoyl-N-(2-methoxycarbonyl-ethyl)-amino]-ethyloxy]-4'-cyano-biphenyl
(13) 4-Cyano-4'-[(methoxycarbonylmethyl-aminocarbonyl)-methyloxy]-biphenyl
(14) 4-Cyano-4'-[N-[(methoxycarbonylmethyl-aminocarbonyl)-methyl]-N-methyl-amino]-biphenyl
(Als Base verwendet man Ethyldiisopropylamin)
(15) 4-Cyano-4'-[N-[[N-(2-methoxycarbonyl-ethyl)-N-methyl-amino]-carbonyl]-N-methyl-amino]-biphenyl
(hergestellt aus 4-Cyano-4'-[[(2-methoxycarbonyl-ethyl)-aminocarbonyl]-amino]-biphenyl durch Methylierung mit Methyljodid).
(16) 4-Cyano-4'-[[(4-methoxycarbonyl-piperidino)-carbonyl]-methyloxy]-biphenyl
(17) 4-Cyano-4'-(5-methoxycarbonyl-pentyloxy)-3'-methylthio]-biphenyl
(18) 4-Cyano-4'-[N-(3-methoxycarbonyl-propionyl)-N-methylamino]-biphenyl
(hergestellt aus 4-Cyano-4'-[(3-methoxycarbonyl-propionyl)-amino]-biphenyl durch Methylierung mit Methyljodid)
(19) 4-Cyano-4'-[N-(4-methoxycarbonyl-butyryl)-N-methylamino]-biphenyl
(hergestellt aus 4-Cyano-4'-[(4-methoxycarbonyl-butyryl)-amino]-biphenyl durch Methylierung mit Methyljodid)
(20) 4-Cyano-4'-[[N-(3-methoxycarbonyl-propyl)-N-methylamino]-sulfonyl]-biphenyl
(hergestellt aus 4-Cyano-4'-[(3-methoxycarbonyl-propyl)-aminosulfonyl]-biphenyl durch Methylierung mit Methyljodid)
(21) 4-Cyano-4'-[N-(4-methoxycarbonyl-butyryl)-N-methylamino]-biphenyl
(hergestellt aus 4-Cyano-4'-(4-methoxycarbonyl-butyrylamino)-biphenyl durch Methylierung mit Methyljodid)
(22) 4-Cyano-4'-[[(2-methoxycarbonyl-ethyl)-thiomethyl]-carbonyl]-biphenyl
(hergestellt aus 4-Bromacetyl-4'-cyano-biphenyl und 3-Mercaptopropionsäuremethylester)
(23) 4-Cyano-4'-[2-(methoxycarbonyl-methylthio)-ethyl]-biphenyl
(hergestellt analog (22))
(24) 4-Cyano-4'-[(3-methoxycarbonyl-propyl)-thiomethyl]-biphenyl
(hergestellt analog (22))

Beispiel 13

4-Cyano-4'-[[(3-ethoxycarbonyl-piperidino)-carbonyl]-methyloxy]-biphenyl

Zu einer Lösung von 7,6 g 4-(Carboxymethyloxy)-4'-cyano-biphenyl (hergestellt aus 4-Cyano-4'-hydroxy-biphenyl und Bromessigsäure-tert.butylester nach Beispiel 13, jedoch mit Kalium-tert.butylat als Base, und anschließender Esterspaltung mit Trifluoressigsäure) in 30 ml Tetrahydrofuran gibt man 5,35 g Carbonyldiimidazol und rührt 0,5 Stunden bei Raumtemperatur. Man fügt 5,1 ml Piperidin-3-carbonsäure-ethyl-ester zu und rührt 22 Stunden bei Raumtemperatur. Das Tetrahydrofuran wird abgedampft, der Rückstand mit Essigester aufgenommen und nacheinander mit gesättigter Natriumbicarbonatlösung, 0,1N Salzsäure und Wasser gewaschen. Nach Eindampfen der organischen Phase hinterbleibt der Rückstand als Öl.
Ausbeute: 10,5 g (89 % der Theorie),

| | | | | | | |
|---|---|---|---|---|---|---|
| Ber.:<br>Gef.: | C | 70,39<br>70,12 | H | 6,16<br>6,45 | N | 7,14<br>7,14 |

Analog werden folgende Verbindungen erhalten:
(1) 4-Cyano-4'-[[(4-ethoxycarbonyl-piperidino)-carbonyl]-methyloxy]-biphenyl
Schmelzpunkt: 98-100 °C
(2) 4-Cyano-4'-[(4-methoxycarbonylmethyl-piperidino)-carbonyl]-biphenyl
Schmelzpunkt: 124-125 °C,
$R_f$-Wert: 0,61 (Kieselgel; Methylenchlorid/Ethanol = 1:1)
(3) 4-Cyano-4'-[(3-ethoxycarbonylmethyl-piperidino)-carbonyl]-biphenyl
(4) 4-Cyano-4'-[(3-methoxycarbonylmethyl-pyrrolidino)-carbonyl]-biphenyl
(5) 4-Cyano-4'-[(4-ethoxycarbonyl-piperidino)-carbonylmethyl]-biphenyl
(6) 4-Cyano-4'-[(3-ethoxycarbonylmethyl-piperidino)-carbonylmethyl]-biphenyl
(7) 4-Cyano-4'-[(3-methoxycarbonylmethyl-pyrrolidino)-carbonylmethyl]-biphenyl
(8) 4-Cyano-4'-[(3-ethoxycarbonyl-piperidino)-carbonylmethyl]-biphenyl
(9) 4-(N-Benzyloxycarbonyl-amidino)-4'-[(4-benzoyloxycarbonylmethyl-4-hydroxy-piperidino)-carbonyl]-biphenyl
(10) 4-Cyano-4'-[(4-methoxycarbonylmethylen-piperidino)-carbonyl]-biphenyl
(11) 4-Cyano-4'-[(4,4-bis-methoxycarbonylmethyl-piperidino)-carbonyl]-biphenyl
(12) 4-Cyano-4'-[(4-sulfomethyl-piperidino)-carbonyl]-biphenyl
(13) 4-Cyano-4'-[[4-(5-tetrazolyl-methyl)-piperidino]-carbonyl]-biphenyl

Beispiel 14

4-Cyano-4'-[(2-ethoxycarbonyl-ethylamino)-carbonyl]-biphenyl

Eine Mischung aus 2 g 4'-Cyano-biphenylyl-4-essigsäure, 1,9 ml N-Methyl-morpholin und 100 ml Tetrahydrofuran wird auf -30 °C abgekühlt und mit 1,1 ml Chlorameisensäure-isobutylester versetzt. Man rührt eine Stunde, fügt 1,3 g β-Alaninethylester-hydrochlorid zu und rührt 50 Stunden bei Raumtemperatur weiter. Die erhaltene Lösung wird in 300 ml 0,5 molare Kaliumhydrogensulfatlösung eingerührt und mit Essigester extrahiert. Die Essigesterphase wird eingeengt und mit Ether versetzt, wobei das Produkt kristallin anfällt.
Ausbeute: 1,1 g (39 % der Theorie),
Schmelzpunkt: 132-136 °C
Analog werden folgende Verbindungen erhalten:
(1) 4-Cyano-4'-[(3-ethoxycarbonyl-propylamino)-carbonyl]-biphenyl
(2) 4-Cyano-4'-[[(2-ethoxycarbonyl-ethylamino)-carbonyl]-biphenyl
(3) 4-Cyano-4'-[[N-(3-methoxycarbonyl-2-propenyl)-N-methylamino]-carbonyl]-biphenyl
(4) 4-[[2-Benzyloxycarbonyl-1-[[2-(4-methoxy-phenyl)-ethyl]-aminocarbonyl]-ethylamino]-carbonylmethyl]-4'-cyano-biphenyl
(5) 4-Cyano-4'-[(4-ethoxycarbonylmethyl-piperidino)-carbonyl]-biphenyl
Schmelzpunkt: 118-120 °C
(6) 4-Cyano-4'-[(4-dimethylaminocarbonylmethyl-piperidino)-carbonyl]-biphenyl

Beispiel 15

4-Cyano-4'-(4-methoxycarbonyl-butyrylamino)-biphenyl

6,6 g 4-Amino-4'-cyano-biphenyl (Schmelzpunkt: 171-173 °C, hergestellt durch Reduktion von 4-Cyano-4'-nitro-biphenyl, welches durch Nitrieren von 4-Cyano-biphenyl mit rauchender Salpetersäure erhalten wird, mit Wasserstoff in Gegenwart von Palladium/Kohle in Essigester) und 5,8 g N-Ethyl-diisopropylamin werden in 70 ml Methylenchlorid gelöst und unter Rühren 5,6 g Glutarsäuremonomethylesterchlorid zugegeben. Man rührt zwei Stunden bei Raumtemperatur weiter. Die organische Phase wird nacheinander mit 0,1N Natronlauge, 0,1N Salzsäure und Wasser gewaschen. Nach Eindampfen der organischen Phase wird der Rückstand aus Ethanol umkristallisiert.
Ausbeute: 7,5 g (68 % der Theorie),
Schmelzpunkt: 153-155 °C
Analog werden folgende Verbindungen erhalten:
(1) 3-Amino-3'-(5-methoxycarbonyl-valerylamino)-biphenyl
(2) 3-Brom-4'-cyano-4-(4-methoxycarbonyl-butyrylamino)-biphenyl
(Diese Verbindung kann auch durch Bromierung von 4-Cyano-4'-(4-methoxycarbonyl-butyrylamino)-biphenyl mit Brom in Eisessig gewonnen werden)
(3) 4-Cyano-3',5'-dibrom-4'-(4-methoxycarbonyl-butyrylamino)-biphenyl
(4) 3-Acetylamino-4'-cyano-4-(5-methoxycarbonyl-pentyloxy)-biphenyl
(5) 3-Benzoylamino-4'-cyano-4-(5-methoxycarbonyl-pentyloxy)-biphenyl
(6) 4-Cyano-3'-methansulfonylamino-4'-(5-methoxycarbonyl-pentyloxy)-biphenyl
(7) 3-Benzolsulfonylamino-4'-cyano-4-(5-methoxycarbonyl-pentyloxy)-biphenyl
(8) 4-Cyano-4'-[(3-methoxycarbonyl-propyl)-aminosulfonyl]-biphenyl
(9) 4-Cyano-4'-methoxy-3'-[(4-methoxycarbonyl-butyl)-aminosulfonyl]-biphenyl
(Hergestellt aus dem intermediär erzeugten 5-Trimethylsilylamino-valeriansäure-trimethylsilylester und anschließende Veresterung mit methanolischer Salzsäure)
$R_f$-Wert: 0,31 (Kieselgel; Methylenchlorid/Methanol = 15:1)

Beispiel 16

4-Cyano-4'-[(4-methoxycarbonyl-butyl)-sulfonyl]-biphenyl

Zu einer Lösung von 4-Cyano-4'-(4-methoxycarbonyl-butylthio)-biphenyl in einem 10:6-Gemisch aus Essigsäureanhydrid und Eisessig wird bei 90 °C langsam 30%iges Wasserstoffperoxid gegeben. Man erhitzt noch eine Stunde weiter, gießt auf Eiswasser, neutralisiert und extrahiert mit Essigester. Die Essigesterphasen werden eingedampft und der Rückstand säulenchromatographisch gereinigt.

Analog werden folgende Verbindungen erhalten:
(1) 4-Cyano-4'-[(3-methoxycarbonyl-propyl)-sulfonylmethyl]-biphenyl
(2) 4-Cyano-4'-(5-methoxycarbonyl-pentyloxy)-3'-methylsulfonyl-biphenyl

Beispiel 17

3-Amino-4'-cyano-4-(5-methoxycarbonyl-pentyloxy)-biphenyl

Hergestellt aus 4-Cyano-4'-(5-methoxycarbonyl-pentyloxy)-3'-nitro-biphenyl durch Hydrierung mit Wasserstoff von 5 bar Druck in Methanol in Gegenwart von 10%iger Palladiumkohle bei Raumtemperatur.

Beispiel 18

4-Cyano-4'-[(4-methoxycarbonylmethyl-piperidino)-methyl]-biphenyl

Hergestellt durch Alkylierung von Piperidyl-4-essigsäure-methylester mit 4-Brommethyl-4'-cyano-biphenyl in Dimethylformamid in Gegenwart von Diisopropylethylamin.

Analog werden folgende Verbindungen erhalten:
(1) 4-Cyano-4'-[(3-methoxycarbonylmethyl-piperidino)-methyl]-biphenyl
(2) 4-Cyano-4'-[2-(4-methoxycarbonyl-piperidino)-ethyl]-biphenyl
(3) 4-Cyano-4'-[2-(3-methoxycarbonylmethyl-piperidino)-ethyl]-biphenyl

(4) 4-Cyano-4'-[(3-methoxycarbonylmethyl-pyrrolidino)-methyl]-biphenyl
(5) 4-Cyano-4'-[2-(3-methoxycarbonylmethyl-pyrrolidino)-ethyl]-biphenyl
(6) 4-Cyano-4'-[2-(3-methoxycarbonyl-piperidino)-ethyl]-biphenyl

Beispiel 19

4-Cyano-4'-[(2-ethoxycarbonylethyl-aminocarbonyl)-amino]-biphenyl

Hergestellt durch Umsetzung von 4-Amino-4'-cyano-biphenyl mit 3-Isocyanato-propionsäure-ethylester in Dioxan bei 50 °C.

Beispiel 20

4-Cyano-4'-[(2-ethoxycarbonyl-ethyl)-aminosulfonylamino]-biphenyl

0,68 g 4-Amino-4'-cyan-biphenyl und 1,1 g 2-[(2-Ethoxycarbonyl-ethyl)-aminosulfonyloxy]-phenol werden in 5 ml Dimethylformamid 15 Stunden auf 80 °C erhitzt. Das Dimethylformamid wird im Vakuum abdestilliert und der Rückstand in Essigester aufgenommen. Nach Waschen mit Wasser wird die organische Phase eingedampft und der verbleibende Rückstand säulenchromatographisch gereinigt (Kieselgel; Elutionsmittel: Cyclohexan/Essigester = 7:3).
Ausbeute: 0,8 g (62 % der Theorie),
$R_f$-Wert: 0,19 (Kieselgel; Cyclohexan/Essigester = 7:3)

Beispiel 21

Trockenampulle mit 2,5 mg Wirkstoff pro 1 ml

Zusammensetzung:

| | |
|---|---|
| Wirkstoff | 2,5 mg |
| Mannitol | 50,0 mg |
| Wasser für Injektionszwecke | ad 1,0 ml |

Herstellung:

Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

Beispiel 22

Trockenampulle mit 35 mg Wirkstoff pro 2 ml

Zusammensetzung:

| | |
|---|---|
| Wirkstoff | 35,0 mg |
| Mannitol | 100,0 mg |
| Wasser für Injektionszwecke | ad 2,0 ml |

Herstellung:

Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

Beispiel 23

Tablette mit 50 mg Wirkstoff

Zusammensetzung:

| | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

Beispiel 24

Tablette mit 350 mg Wirkstoff

Zusammensetzung:

| | |
|---|---|
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

Beispiel 25

Kapseln mit 50 mg Wirkstoff

Zusammensetzung:

| | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2,0 mg |
| | 160,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

Beispiel 26

Kapseln mit 350 mg Wirkstoff

Zusammensetzung:

| | |
|---|---|
| (1) Wirkstoff | 300,0 mg |
| (2) Maisstärke getrocknet | 46,0 mg |
| (3) Milchzucker pulverisiert | 30,0 mg |
| (4) Magnesiumstearat | 4,0 mg |
| | 430,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

## Patentansprüche

**1.** Biphenylderivate der allgemeinen Formel

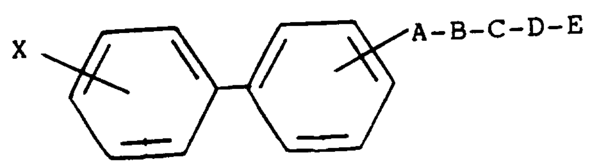

,(I)

in der

der mit dem Rest X verbundene Phenylring durch ein Fluor-, Chlor- oder Bromatom,

der mit dem Rest A verbundene Phenylring durch ein Fluor- oder Chloratom, eine Hydroxy-, Methoxy-, Trifluormethyl-, Methylsulfenyl-, Methylsulfinyl-, Methylsulfonyl-, Nitro-, Amino-, Acetylamino-, Benzoylamino-, Methansulfonylamino- oder Benzolsulfonylaminogruppe oder durch eine oder zwei Methylgruppen oder durch ein oder zwei Bromatome substituiert sein kann,

X eine Aminomethyl-, Amidino- oder Guanidinogruppe, in denen ein Wasserstoffatom an einem der Stickstoffatome durch eine Alkylgruppe mit 1 bis 4 Kohlenstoffatomen, durch eine Methoxycarbonyl-, Ethoxycarbonyl- oder Benzyloxycarbonylgruppe ersetzt sein kann, oder eine Cyanogruppe,

A eine Bindung, ein Sauerstoff- oder Schwefelatom, eine -NH-CO-, $-NCH_3-CO-$, -CO-NH-, $-CO-NCH_3-$, $-NCH_3-$, -SO-, $-SO_2-$, $-SO_2-NH-$, $-SO_2-NCH_3-$, -CO-, -NH-CO-NH-, $-NH-SO_2-NH-$ oder $-NCH_3-CO-NCH_3-$Gruppe,

B eine Bindung, eine geradkettige oder verzweigte Alkylengruppen mit 1 bis 5 Kohlenstoffatomen, eine geradkettige oder verzweigte Alkenylengruppe mit 3 Kohlenstoffatomen, wobei die Doppelbindung nicht direkt mit einem Sauerstoff-, Schwefel- oder Phosphoratom der Reste A, C oder E verbunden sein kann, eine Cyclohexylen- oder Phenylengruppe,

C eine Bindung oder, sofern eine CO-Gruppe des Restes C nicht unmittelbar auf ein Sauerstoff- oder Schwefelatom oder eine -CO-NH- oder $-CO-NCH_3$-Gruppe des Restes A folgt, eine -CO-NH-, $-CO-NCH_3-$, $-CO-NH-(CH_2)_2-CH(CH_2-COOH)-$, $-CO-NCH_3-(CH_2)_2-CH(CH_2-COOH)-$, $-CO-NH-(CH_2)_2-CH(CH_2-COOCH_3)-$, $-CO-NCH_3-(CH_2)_2-CH(CH_2-COOCH_3)-$, Pyrrolidinylen-N-carbonyl-, Piperidinylen-N-carbonyl-, Piperazinylen-N-carbonyl- oder 4-Methanylyliden-piperidinocarbonyl-gruppe, wobei die Gruppe -D-E an die Methanylylidengruppe gebunden ist, eine 4-Hydroxy-4-piperidinylen-N-carbonyl-, 4-Carboxymethyl-4-piperidinylen-N-carbonyl- oder 4-Methoxycarbonylmethyl-4-piperidinylen-N-carbonyl-gruppe, wobei die Gruppe -D-E an die 4-Stellung gebunden ist, oder eine [[[2-(Methoxyphenyl)-ethyl]-aminocarbonyl]-methylen]-aminocarbonyl-Gruppe, wobei die Gruppe -D-E an das Methylenkohlenstoffatom gebunden ist,
D eine Bindung, eine geradkettige oder verzweigte Alkylengruppe mit 1 bis 3 Kohlenstoffatomen oder eine Alkylenphenylengruppe mit 1 oder 2 Kohlenstoffatomen im Alkylenteil und
E eine Carboxy-, Sulfo-, Phosphono-, 5-Tetrazolyl- oder O-Methyl-phosphonogruppe, eine Alkoxycarbonylgruppe mit insgesamt 2 bis 5 Kohlenstoffatomen, in der der Alkoxyteil in 1- oder 2-Stellung durch eine Phenylgruppe substituiert sein kann, eine Aminocarbonyl-, Methylaminocarbonyl- oder Dimethylaminocarbonylgruppe, bedeuten, wobei mindestens einer der Reste A, B, C oder D keine Bindung darstellt, der Rest E nicht unmittelbar auf ein Heteroatom der Reste A oder C folgen kann, und, sofern X eine Aminomethylgruppe darstellt, der kürzeste Abstand zwischen der Aminogruppe und dem Rest E mindestens 12 Bindungen beträgt,
deren Stereoisomere, einschließlich ihrer Gemische und deren Salze.

**2.** Biphenylderivate der allgemeinen Formel I gemäß Anspruch 1, in denen
der mit dem Rest X verbundene Phenylring unsubstituiert ist und der mit dem Rest A verbundene Phenylring durch eine Hydroxy- oder Methoxygruppe substituiert sein kann,
X eine Aminomethyl- oder Amidinogruppe, in denen ein Wasserstoffatom an einem der Stickstoffatome durch eine Methoxycarbonyl-, Ethoxycarbonyl- oder Benzyloxycarbonylgruppe ersetzt sein kann,
A eine Bindung, ein Sauerstoffatom, eine -NH-CO-, -CO-NH-, $-CO-NCH_3-$, $-SO_2-NH-$ oder $-NH-SO_2-NH-$Gruppe,
B eine Bindung, eine geradkettige Alkylengruppe mit 1 bis 5 Kohlenstoffatomen, eine Cyclohexylen- oder Phenylengruppe,
C eine Bindung oder, sofern C nicht unmittelbar auf ein Sauerstoffatom oder eine -CO-NH- oder $-CO-NCH_3$-Gruppe des Restes A folgt, eine -CO-NH-Gruppe, eine Piperidinylen-N-carbonylgruppe, die in 3- oder 4-Stellung mit der Gruppe -D-E verknüpft ist, eine 4-Piperazinylen-N-carbonyl- oder 4-Methanylyliden-piperidinocarbonylgruppe, wobei die Gruppe -D-E an die Methanylylidengruppe gebunden ist, eine 4-Hydroxy-4-piperidinylen-N-carbonyl-, 4-Carboxymethyl-4-piperidinylen-N-carbonyl- oder 4-Methoxycarbonylmethylen-piperidinylen-N-carbonylgruppe, wobei der Rest -D-E an die 4-Stellung gebunden ist, oder eine [[[2-(4-Methoxyphenyl)-ethyl]-aminocarbonyl]-methylen]-aminocarbonylgruppe, wobei die Gruppe -D-E an das Methylenkohlenstoffatom geknüpft ist,
D eine Bindung, eine Methylen-, Ethylen-, Methylenphenylen- oder Ethylenphenylengruppe und
E eine Carboxyl-, Methoxycarbonyl-, Ethoxycarbonyl-, Benzyloxycarbonyl-, Aminocarbonyl-, Dimethylaminocarbonyl- oder 5-Tetrazolylgruppe bedeuten, wobei mindestens einer der Reste A, B, C oder D keine Bindung darstellt und E nicht unmittelbar auf ein Heteroatom der Reste A oder C folgen kann, und, sofern X eine Aminomethylgruppe darstellt, der kürzeste Abstand zwischen der Aminogruppe und dem Rest E mindestens 12 Bindungen beträgt,
deren Stereoisomere, einschließlich ihrer Gemische und deren Salze.

**3.** Biphenylderivate der allgemeinen Formel I gemäß Anspruch 1, in denen
der Biphenylylrest unsubstituiert ist,
X eine Aminomethyl- oder Amidinogruppe, in denen ein Wasserstoffatom an einem der Stickstoffatome durch eine Methoxycarbonyl- oder Benzyloxycarbonylgruppe ersetzt sein kann,
A eine Bindung, ein Sauerstoffatom, eine -NH-CO- oder -CO-NH-Gruppe,
B eine Bindung, eine geradkettige Alkylengruppe mit 1 bis 5 Kohlenstoffatomen oder eine Cyclohexylengruppe,
C eine Bindung oder, sofern C nicht unmittelbar auf ein Heteroatom oder eine Carbonylgruppe des Restes A folgt, eine -CO-NH-Gruppe, eine Piperidinylen-N-carbonylgruppe, die in 3- oder 4-Stellung mit der Gruppe -D-E verknüpft ist, eine Piperazinylen-N-carbonylgruppe, wobei der Rest -D-E an die 4-Stellung gebunden ist, oder eine [[[2-(4-Methoxyphenyl)-ethyl]-aminocarbonyl]-methylen]-aminocarbonylgruppe, wobei die Gruppe -D-E an das Methylenkohlenstoffatom geknüpft ist,

D eine Bindung, eine Methylen- oder Ethylengruppe und
E eine Carboxyl-, Methoxycarbonyl-, Ethoxycarbonyl- oder Benzyloxycarbonylgruppe bedeuten, wobei mindestens einer der Reste A, B, C oder D keine Bindung darstellt und E nicht unmittelbar auf ein Heteroatom der Reste A oder C folgen kann, und, sofern X eine Aminomethylgruppe darstellt, der kürzeste Abstand zwischen der Aminogruppe und dem Rest E mindestens 12 Bindungen beträgt,
deren Stereoisomere, einschließlich ihrer Gemische und deren Salze.

4. Folgende Verbindungen der allgemeinen Formel I gemäß Anspruch 1:
4-Amidino-4'-[(4-carboxymethyl-piperidino)-carbonyl]-biphenyl,
4-Amidino-4'-[(4-carboxymethyl-piperazino)-carbonyl]-biphenyl,
4-Amidino-4'-[(4-carboxy-cyclohexyl)-aminocarbonyl]-biphenyl,
4-Amidino-4'-[(4-methoxycarbonylmethyl-piperidino)-carbonyl]-biphenyl,
4-Amidino-4'-[(4-methoxycarbonyl-cyclohexyl)-aminocarbonyl]-biphenyl,
4-(N-Methoxycarbonyl-amidino)-4'-[(4-methoxycarbonylmethyl-piperidino)-carbonyl]-biphenyl,
4-Amidino-3'-[(4-carboxy-cyclohexyl)-aminocarbonyl]-4'-methoxy-biphenyl,
4-Aminomethyl-3'-[(4-carboxy-cyclohexyl)-aminocarbonyl]-4'-methoxy-biphenyl und
4-Amidino-3'-[N-(4-carboxy-cyclohexyl)-N-methyl-aminocarbonyl]-4'-hydroxy-biphenyl,
deren Stereoisomere, einschließlich ihrer Gemische und deren Salze.

5. Physiologisch verträgliche Additionssalze der Verbindungen nach mindestens einem der Ansprüche 1 bis 4 mit anorganischen oder organischen Säuren oder Basen.

6. Arzneimittel, enthaltend eine Verbindung nach mindestens einem der Ansprüche 1 bis 4 oder ein physiologisch verträgliches Additionssalz gemäß Anspruch 5 neben gegebenenfalls einem oder mehreren inerten Trägerstoffen und/oder Verdünnungsmitteln.

7. Verwendung einer Verbindung nach mindestens einem der Ansprüche 1 bis 5 zur Herstellung eines Arzneimittels, das zur Bekämpfung bzw. Verhütung von Krankheiten, bei denen kleinere oder größere Zell-Aggregate auftreten oder Zell-Matrixinteraktionen eine Rolle spielen, geeignet ist.

8. Verfahren zur Herstellung eines Arzneimittels gemäß Anspruch 6, dadurch gekennzeichnet, daß auf nichtchemischem Wege eine Verbindung nach mindestens einem der Ansprüche 1 bis 5 in einen oder mehrere inerte Trägerstoffe und/oder Verdünnungsmittel eingearbeitet wird.

9. Verfahren zur Herstellung der Biphenylderivate gemäß den Ansprüchen 1 bis 5, dadurch gekennzeichnet, daß

a) zur Herstellung von Verbindungen der allgemeinen Formel I, in der X eine gegebenenfalls durch eine Alkylgruppe mit 1 bis 4 Kohlenstoffatomen substituierte Amidinogruppe darstellt, eine gegebenenfalls im Reaktionsgemisch gebildete Verbindung der allgemeinen Formel

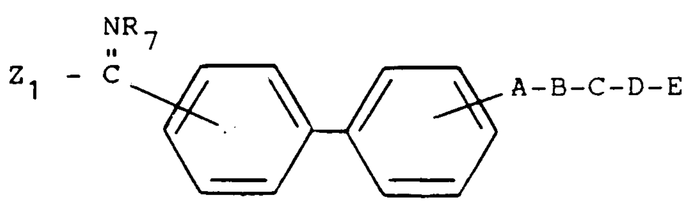

,(II)

in der
A, B, C, D und E wie in den Ansprüchen 1 bis 4 definiert sind,
$R_7$ ein Wasserstoffatom oder eine Alkylgruppe mit 1 bis 4 Kohlenstoffatomen und
$Z_1$ eine Alkoxy-, Aralkoxy-, Alkylthio-, Aralkylthio- oder Aminogruppe darstellen, mit einem Amin der allgemeinen Formel

$NH_2 - R_8$ ,(III)

in der

$R_8$ ein Wasserstoffatom oder eine Alkylgruppe mit 1 bis 4 Kohlenstoffatomen bedeutet, oder mit deren Säureadditionssalzen umgesetzt wird oder

b) zur Herstellung von Verbindungen der allgemeinen Formel I, in der X eine Aminomethylengruppe darstellt, eine Verbindung der allgemeinen Formel

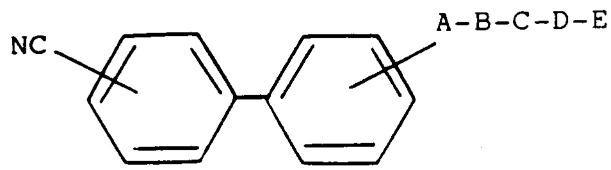

,(IV)

in der

A, B, C, D und E wie in den Ansprüchen 1 bis 4 definiert sind, reduziert wird oder

c) zur Herstellung von Verbindungen der allgemeinen Formel I, in der X eine Guanidinogruppe darstellt, eine Verbindung der allgemeinen Formel

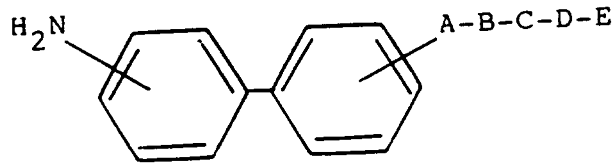

,(V)

in der

A, B, C, D und E wie in den Ansprüchen 1 bis 4 definiert sind, oder dessen Säureadditionssalz mit Cyanamid umgesetzt wird oder

d) zur Herstellung von Verbindungen der allgemeinen Formel I, in der E eine Carboxylgruppe darstellt, eine Verbindung der allgemeinen Formel

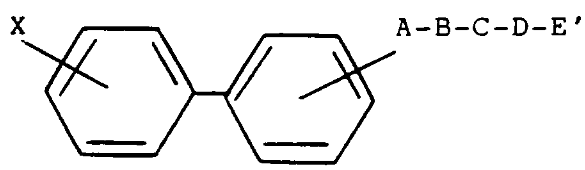

,(VI)

in der

A, B, C, D und X wie in den Ansprüchen 1 bis 4 definiert sind und

E', das an ein Kohlenstoffatom gebunden ist, eine mittels Hydrolyse, Behandlung mit Säuren, Thermolyse oder Hydrogenolyse in eine Carboxyl- oder Bis(hydroxycarbonyl)methylgruppe überführbare Gruppe darstellt, in eine entsprechende Verbindung umgewandelt und erforderlichenfalls anschließend eine so erhaltene Bis(hydroxycarbonyl)methyl-Verbindung decarboxyliert wird oder

e) zur Herstellung von Verbindungen der allgemeinen Formel I, in der A eine -NH-CO-, $-NCH_3-CO-$, -CO-NH-, $-CO-NCH_3-$, $-SO_2-NH-$ oder $-SO_2-NCH_3$-Gruppe darstellt, eine Verbindung der allgemeinen Formel

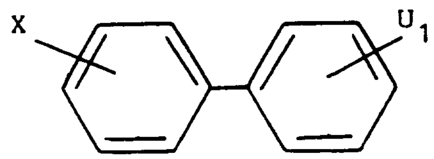

,(VII)

mit einer Verbindung der allgemeinen Formel

$U_2$ - C - D - E ,(VIII)

in denen
C, D, E und X wie in den Ansprüchen 1 bis 4 definiert sind, einer der Reste $U_1$ oder $U_2$ eine $HNR_3$-Gruppe, wobei
$R_3$ ein Wasserstoffatom oder eine Methylgruppe darstellt, und
der andere der Reste $U_1$ oder $U_2$ eine $Z_2$-A'-Gruppe bedeuten, wobei
A' eine Carbonyl- oder Sulfonylgruppe und
$Z_2$ eine Hydroxygruppe oder eine nukleophile Austrittsgruppe darstellen,
oder mit deren reaktionsfähigen Derivaten umgesetzt wird oder
f) zur Herstellung von Verbindungen der allgemeinen Formel I, in der die E-D-C-Gruppe eine HOOC-$(CH_2)_n$-CO-NH-Gruppe darstellt, eine Verbindung der allgemeinen Formel

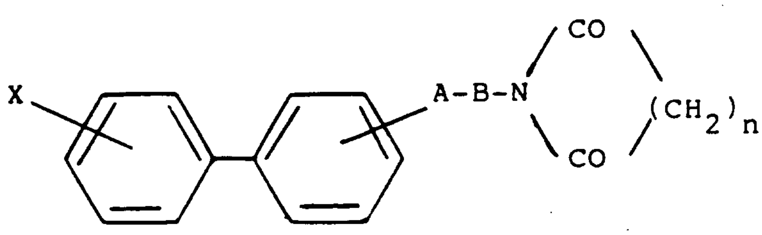

,(IX)

in der
A, B, X und n wie in den Ansprüchen 1 bis 4 definiert sind, hydrolysiert wird oder
g) zur Herstellung von Verbindungen der allgemeinen Formel I, in der E eine Alkoxcarbonylgruppe mit insgesamt 2 bis 5 Kohlenstoffatomen, in der der Alkoxyteil in 1- oder 2-Stellung durch eine Phenylgruppe substituiert sein kann, darstellt, eine Verbindung der allgemeinen Formel

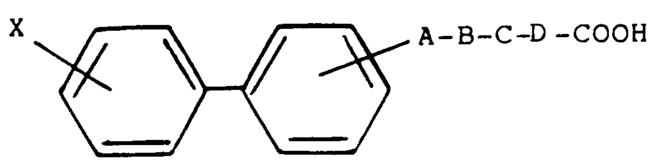

,(X)

in der
A, B, C, D und X wie in den Ansprüchen 1 bis 4 definiert sind, oder deren reaktionsfähige Derivate, mit einer Verbindung der allgemeinen Formel

H - $R_9$ ,(XI)

in der
$R_9$ eine Alkoxygruppe mit 1 bis 4 Kohlenstoffatomen, in der der Alkoxyteil in 1- oder 2-Stellung durch eine Phenylgruppe substituiert sein kann, darstellt, umgesetzt wird oder
h) zur Herstellung von Verbindungen der allgemeinen Formel I, in der die Gruppe X eine Methoxycarbonyl-, Ethoxycarbonyl- oder Benzyloxycarbonylgruppe enthält, eine Verbindung der allgemei-

nen Formel

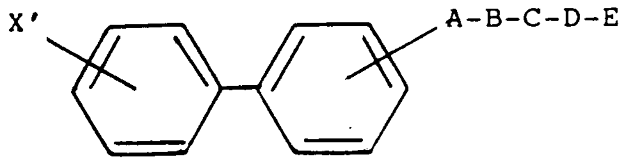

,(XII)

in der
A, B, C, D und E wie in den Ansprüchen 1 bis 4 definiert sind und
X' eine Aminomethyl-, Amidino- oder Guanidinogruppe darstellt, mit einem Ester der allgemeinen Formel

$Z_3$ - CO - $OR_3$' ,(XIII)

in der
$R_3$' eine Methyl-, Ethyl- oder Benzylgruppe und
$Z_3$ eine nukleophile Austrittsgruppe darstellen, umgesetzt wird oder
i) zur Herstellung von Verbindungen der allgemeinen Formel I, in der A oder C eine Sulfinyl- oder Sulfonylgruppe oder der mit dem Rest A verbundene Phenylring eine Methylsulfinyl- oder Methylsulfonylgruppe enthalten, eine Verbindung der allgemeinen Formel

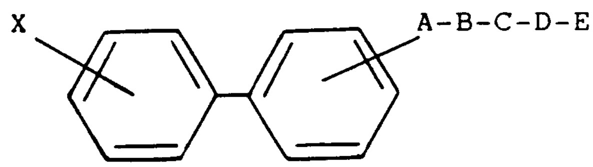

,(XIV)

in der
A, B, C, D, E und X mit der Maßgabe wie in den Ansprüchen 1 bis 4 definiert sind, daß A oder C ein Schwefelatom oder eine Sulfinylgruppe oder der mit dem Rest A verbundene Phenylring eine Methylsulfenyl- oder Methylsulfinylgruppe enthalten, oxidiert wird und
erforderlichenfalls ein während der Umsetzungen a) bis i) zum Schutze von reaktiven Gruppen verwendeter Schutzrest abgespalten wird und/oder
gewünschtenfalls eine so erhaltene Verbindung der allgemeinen Formel I in ihre cis-/trans-Isomere, in ihre Enantiomeren und/oder Diastereomeren aufgetrennt wird und/oder
eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

**Claims**

1. Biphenyl derivatives of general formula

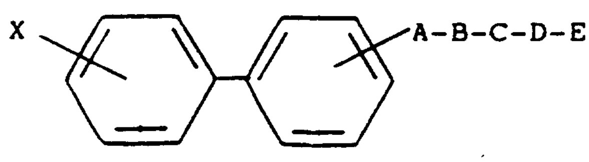

,(I)

wherein

the phenyl group linked to the group X may be substituted by a fluorine, chlorine or bromine atom,
the phenyl ring linked to the group A may be substituted by a fluorine or chlorine atom or by a hydroxy, methoxy, trifluoromethyl, methylsulphenyl, methylsulphinyl, methylsulphonyl, nitro, amino, acetylamino, benzoylamino, methanesulphonylamino or benzenesulphonylamino group or by one or two methyl groups or by one or two bromine atoms,
X represents an aminomethyl, amidino or guanidino group wherein a hydrogen atom at one of the nitrogen atoms may be replaced by a $C_{1-4}$-alkyl group, by a methoxycarbonyl, ethoxycarbonyl or benzyloxycarbonyl group, or a cyano group
A represents a bond, an oxygen or sulphur atom, an -NH-CO, $-NCH_3-CO$, -CO-NH, $-CO-NCH_3$, $-NCH_3$, -SO, $-SO_2$, $-SO_2-NH$, $-SO_2-NCH_3$, -CO, -NH-CO-NH, $-NH-SO_2-NH$ or $-NCH_3-CO-NCH_3$ group,
B represents a bond, a straight-chained or branched $C_{1-5}$-alkylene group, a straight-chained or branched $C_3$-alkenylene group, whilst the double bond cannot be directly linked to an oxygen, sulphur or phosphorus atom of groups A, C or E, or a cyclohexylene or phenylene group,
C represents a bond or, if a CO group of the group C does not directly follow an oxygen or sulphur atom or a -CO-NH or $-CO-NCH_3$ group of the group A, a -CO-NH, $-CO-NCH_3$, $-CO-NH-(CH_2)_2-CH(CH_2-COOH)$, $-CO-NCH_3-(CH_2)_2-CH(CH_2-COOH)$, $-CO-NH-(CH_2)_2-CH(CH_2-COOCH_3)$, $-CO-NCH_3-(CH_2)_2-CH(CH_2-COOCH_3)$, pyrrolidinylene-N-carbonyl, piperidinylene-N-carbonyl, piperazinylene-N-carbonyl or 4-methanylylidene-piperidinocarbonyl group, wherein the group -D-E is bound to the methanylylidene group, a 4-hydroxy-4-piperidinylene-N-carbonyl, 4-carboxymethyl-4-piperidinylene-N-carbonyl or 4-methoxycarbonylmethyl-4-piperidinylene-N-carbonyl group, wherein the group -D-E is bound to the 4-position, or a [[[2-(methoxyphenyl)-ethyl]-aminocarbonyl]-methylene]-aminocarbonyl group, wherein the group -D-E is bound to the methylenecarbon atom,
D represents a bond, a straight-chained or branched $C_{1-3}$-alkylene group or an alkylenephenylene group having 1 or 2 carbon atoms in the alkylene moiety and
E represents a carboxy, sulpho, phosphono, 5-tetrazolyl or O-methyl-phosphono group, an alkoxycarbonyl group having a total of 2 to 5 carbon atoms wherein the alkoxy moiety may be substituted in the 1- or 2-position by a phenyl group, or an aminocarbonyl, methylaminocarbonyl or dimethylaminocarbonyl group, whilst at least one of the groups A, B, C or D does not represent a bond, the group E cannot directly follow a heteroatom of groups A or C and, if X represents an aminomethyl group, the shortest distance between the amino group and the group E is at least 12 bonds,
the stereoisomers thereof, including the mixtures and salts thereof.

**2.** Biphenyl derivatives of general formula I according to claim 1 wherein
the phenyl ring connected to the group X is unsubstituted and the phenyl ring connected to the group A may be substituted by a hydroxy or methoxy group,
X represents an aminomethyl or amidino group in which a hydrogen atom at one of the nitrogen atoms may be replaced by a methoxycarbonyl, ethoxycarbonyl or benzyloxycarbonyl group,
A represents a bond, an oxygen atom, an -NH-CO, -CO-NH, $-CO-NCH_3$, $-SO_2-NH$ or $-NH-SO_2-NH$ group,
B represents a bond, a straight-chained $C_{1-5}$-alkylene group, a cyclohexylene or phenylene group,
C represents a bond or, if C does not directly follow an oxygen atom or a -CO-NH or $-CO-NCH_3$ group of group A, it may represent a -CO-NH group, a piperidinylene-N-carbonyl group linked in the 3- or 4-position to the group -D-E, a 4-piperazinylene-N-carbonyl or 4-methanylylidene-piperidinocarbonyl group, whilst the group -D-E is bound to the methanylylidene group, a 4-hydroxy-4-piperidinylene-N-carbonyl, 4-carboxymethyl-4-piperidinylene-N-carbonyl or 4-methoxycarbonylmethylene-piperidinylene-N-carbonyl group, whilst the group -D-E is bound to the 4-position, or a [[[2-(4-methoxyphenyl)-ethyl]-aminocarbonyl]-methylene]-aminocarbonyl group, whilst the group -D-E is linked to the methylene carbon atom,
D represents a bond, a methylene, ethylene, methylenephenylene or ethylenephenylene group and
E represents a carboxyl, methoxycarbonyl, ethoxycarbonyl, benzyloxycarbonyl, aminocarbonyl, dimethylaminocarbonyl or 5-tetrazolyl group, whilst at least one of the groups A, B, C or D does not represent a bond and E cannot directly follow a heteroatom of groups A or C, and, if X represents an aminomethyl group, the shortest distance between the amino group and group E is at least 12 bonds,
the stereoisomers thereof, including their mixtures and the salts thereof.

**3.** Biphenyl derivatives of general formula I according to claim 1, wherein
the biphenylyl group is unsubstituted,
X is an aminomethyl or amidino group in which a hydrogen atom at one of the nitrogen atoms may be

replaced by a methoxycarbonyl or benzyloxycarbonyl group,
A represents a bond, an oxygen atom, an -NH-CO or -CO-NH group,
B represents a bond, a straight-chained $C_{1-5}$-alkylene group or a cyclohexylene group,
C represents a bond or, if C does not directly follow a heteroatom or a carbonyl group of group A, it may represent a -CO-NH group, a piperidinylene-N-carbonyl group linked in the 3- or 4-position to the group -D-E, a piperazinylene-N-carbonyl group, wherein the group -D-E is bound to the 4-position, or a [[[2-(4-methoxyphenyl)-ethyl]-aminocarbonyl]-methylene]-aminocarbonyl group, wherein the group -D-E is linked to the methylene carbon atom,
D represents a bond, a methylene or ethylene group and
E represents a carboxyl, methoxycarbonyl, ethoxycarbonyl or benzyloxycarbonyl group whilst at least one of the groups A, B, C or D does not represent a bond and E cannot directly follow a heteroatom of group A or C, and if X represents an aminomethyl group the shortest distance between the amino group and the group E is at least 12 bonds,
the stereoisomers, including the mixtures thereof and the salts thereof.

4. The following compounds of general formula I according to claim 1:
4-amidino-4'-[(4-carboxymethyl-piperidino)-carbonyl]-biphenyl,
4-amidino-4'-[(4-carboxymethyl-piperazino)-carbonyl]-biphenyl,
4-amidino-4'-[(4-carboxy-cyclohexyl)-aminocarbonyl]-biphenyl,
4-amidino-4'-[(4-methoxycarbonylmethyl-piperidino)-carbonyl]-biphenyl,
4-amidino-4'-[(4-methoxycarbonyl-cyclohexyl)-aminocarbonyl]-biphenyl,
4-(N-methoxycarbonyl-amidino)-4'-[(4-methoxycarbonyl-methyl-piperidino)-carbonyl]-biphenyl,
4-amidino-3'-[(4-carboxy-cyclohexyl)-aminocarbonyl]-4'-methoxy-biphenyl,
4-aminomethyl-3'-[(4-carboxy-cyclohexyl)-aminocarbonyl]-4-'-methoxy-biphenyl and
4-amidino-3'-[N-(4-carboxy-cyclohexyl)-N-methylaminocarbonyl]-4'-hydroxy-biphenyl,
the stereoisomers, including the mixtures thereof and the salts thereof.

5. Physiologically acceptable addition salts of the compounds according to at least one of claims 1 to 4 with organic or inorganic acids or bases.

6. Pharmaceutical compositions containing a compound according to at least one of claims 1 to 4 or a physiologically acceptable addition salt according to claim 5 optionally together with one or more inert carriers and/or diluents.

7. Use of a compound according to at least one of claims 1 to 5 for preparing a pharmaceutical composition which is suitable for treating or preventing diseases in which smaller or larger cell aggregates occur or in which cell matrix interactions play a part.

8. Process for preparing a pharmaceutical composition according to claim 6, characterised in that a compound according to at least one of claims 1 to 5 is incorporated in one or more inert carriers and/or diluents by a non-chemical method.

9. A process for preparing the biphenyl derivatives according to claims 1 to 5, characterised in that
a) in order to prepare compounds of general formula I wherein X contains an amidino group optionally substituted by a $C_{1-4}$ alkyl group, a compound of general formula

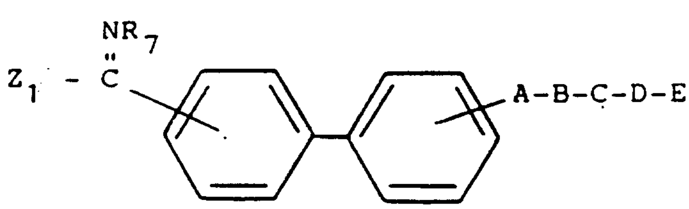

,(II)

optionally formed in the reaction mixture, wherein A, B, C, D and E are defined as in claims 1 to 4, $R_7$ represents a hydrogen atom or a $C_{1-4}$-alkyl group and $Z_1$ represents an alkoxy, aralkoxy, alkylthio, aralkylthio or amino group, is reacted with an amine of general formula

$NH_2 - R_8$ ,(III)

wherein
$R_8$ represents a hydrogen atom or a $C_{1-4}$-alkyl group, or with the acid addition salts thereof or
b) in order to prepare compounds of general formula I wherein X represents an aminomethylene group, a compound of general formula

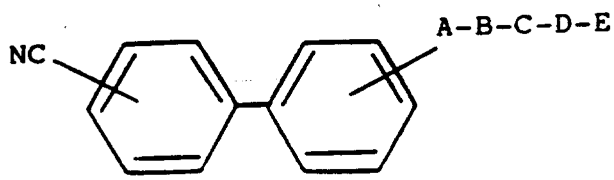

,(IV)

wherein
A, B, C, D and E are defined as in claims 1 to 4, is reduced or
c) in order to prepare compounds of general formula I wherein X represents a guanidino group, a compound of general formula

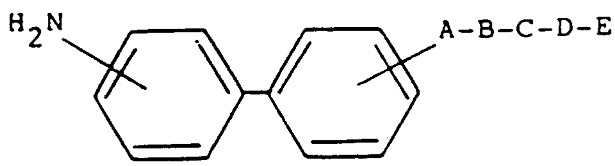

,(V)

wherein
A, B, C, D and E are defined as in claims 1 to 4, or an acid addition salt thereof, is reacted with cyanamide or
d) in order to prepare compounds of general formula I wherein E represents a carboxyl group, a compound of general formula

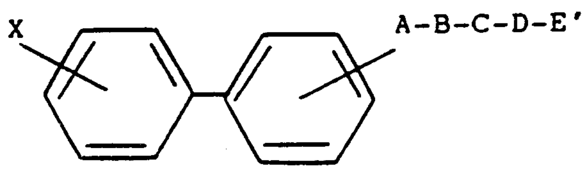

,(VI)

wherein
A, B, C, D and X are defined as in claims 1 to 4 and E', which is bound to a carbon atom, represents a group which may be converted into a carboxyl- or bis-(hydroxycarbonyl)methyl group by hydrolysis, treatment with acids, thermolysis or hydrogenolysis is converted into such a compound and subsequently, if desired, a bis-(hydroxycarbonyl)methyl compound thus obtained is decarboxylated or
e) in order to prepare compounds of general formula I wherein A represents an -NH-CO, $-NCH_3$-CO, -CO-NH, $-CO-NCH_3$, $-SO_2$-NH or $-SO_2-NCH_3$ group, a compound of general formula

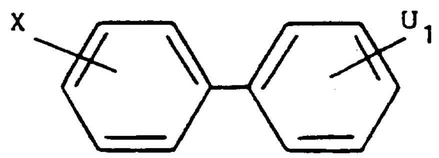

,(VII)

is reacted with a compound of general formula

$U_2$ - C - D - E ,(VIII)

wherein

C, D, E and X are defined as in claims 1 to 4, one of the groups $U_1$ or $U_2$ represents an $HNR_3$ group, wherein $R_3$ is defined as in claims 1 to 4, and the other group $U_1$ or $U_2$ represents a $Z_2$-A' group, wherein

A' represents a carbonyl or sulphonyl group and

$Z_2$ represents a hydroxy group or a nucleophilic leaving group,

or with the reactive derivatives thereof, or

f) in order to prepare compounds of general formula I wherein the E-D-C group represents an HOOC-$(CH_2)_n$-CO-NH group, a compound of general formula

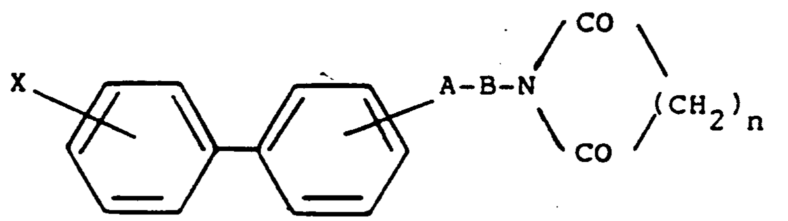

(IX)

wherein

A, B, X and n are defined as in claims 1 to 4, is hydrolysed, or

g) in order to prepare compounds of general formula I wherein E represents an alkoxycarbonyl group with a total of 2 to 5 carbon atoms, wherein the alkoxy moiety may be substituted in the 1- or 2-position by a phenyl group,

a compound of general formula

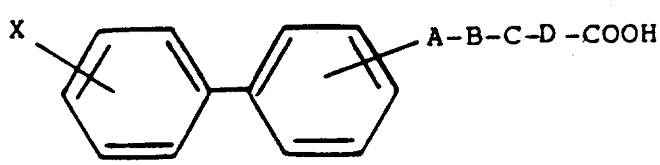

,(X)

wherein

A, B, C, D and X are defined as in claims 1 to 4, or the reactive derivatives thereof, is reacted with a compound of general formula

H - $R_9$ ,(XI)

wherein

$R_9$ represents a $C_{1-4}$-alkoxy group wherein the alkoxy part may be substituted in the 1- or 2-position by a phenyl group, or

h) in order to prepare compounds of general formula I wherein the group X contains a methoxycarbonyl, ethoxycarbonyl or benzyloxycarbonyl group, a compound of general formula

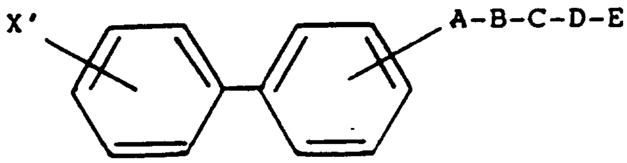

,(XII)

wherein
A, B, C, D and E are defined as in claims 1 to 4 and
X' represents an aminomethyl, amidino or guanidino group, is reacted with an ester of general formula

$Z_3$ - CO - $OR_3$' ,(XIII)

wherein
$R_3$' represents a methyl, ethyl or benzyl group and
$Z_3$ represents a nucleophilically exchangeable group, or
i) in order to prepare compounds of general formula I wherein A or C contains a sulphinyl or sulphonyl group or the phenyl ring attached to the group A contains a methylsulphynyl or methylsulphonyl group, a compound of general formula

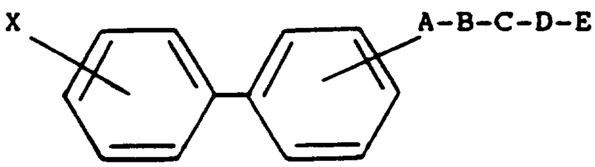

,(XIV)

wherein
A, B, C, D, E and X are defined as in claims 1 to 4, with the proviso that A or C contains a sulphur atom or a sulphynyl group or the phenyl ring attached to the group A contains a methylsulphenyl or methylsulphynyl group, is oxidised and
if required, any protecting group used during reactions a) to i) to protect the reactive groups is cleaved and/or
if desired, a compound of general formula I thus obtained is resolved into the cis/trans isomers, enantiomers and/or diastereomers thereof and/or
a compound of general formula I thus obtained is converted into the salts thereof, more particularly for pharmaceutical use into the physiologically acceptable salts thereof with an organic or inorganic acid or base.

## Revendications

**1.** Dérivés biphényliques de formule générale

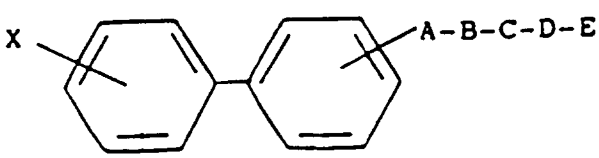

(I)

dans laquelle
le cycle phényle lié au reste X peut être substitué par un atome de fluor, de chlore ou de brome,

le cycle phényle lié au reste A peut être substitué par un atome de fluor ou de chlore, un groupe hydroxyle, méthoxy, trifluorométhyle, méthylsulfényle, méthylsulfinyle, méthylsulfonyle, nitro, amino, acétylamino, benzoylamino, méthanesulfonylamino ou benzènesulfonylamino ou par un ou deux groupes méthyle ou par un ou deux atomes de brome,

X représente un groupe aminométhyle, amidino ou guanidino, dans lesquels un atome d'hydrogène sur l'un des atomes d'azote peut être remplacé par un groupe alkyle de 1 à 4 atomes de carbone, par un groupe méthoxycarbonyle, éthoxycarbonyle ou benzyloxycarbonyle, ou un groupe cyano,

A représente une liaison, un atome d'oxygène ou de soufre, un groupe -NH-CO-, $-NCH_3$-CO-, -CO-NH-, -CO-$NCH_3$-, -$NCH_3$-, -SO-, -$SO_2$-, -$SO_2$-NH-, -$SO_2$-$NCH_3$-, -CO-, -NH-CO-NH-, -NH-$SO_2$-NH- ou -$NCH_3$-CO-$NCH_3$-,

B représente une liaison, un groupe alkylène linéaire ou ramifié de 1 à 5 atomes de carbone, un groupe alcénylène linéaire ou ramifié de 3 atomes de carbone, auquel cas la double liaison ne peut pas être liée directement à un atome d'oxygène, de soufre ou de phosphore des restes A, C ou E, un groupe cyclohexylène ou phénylène,

C représente une liaison, ou, dans la mesure où un groupe CO du reste C ne suit pas immédiatement un atome d'oxygène ou de soufre ou un groupe -CO-NH- ou -CO-$NCH_3$- du reste A, un groupe -CO-NH-, -CO-$NCH_3$-, -CO-NH-$(CH_2)_2$-CH($CH_2$-COOH)-, -CO-$NCH_3$-$(CH_2)_2$-CH($CH_2$-COOH)-, -CO-NH-$(CH_2)_2$-CH($CH_2$-$COOCH_3$)-, -CO-$NCH_3$-$(CH_2)_2$-CH($CH_2$-$COOCH_3$)-, pyrrolidinylène-N-carbonyle, pipéridinylène-N-carbonyle, pipérazinylène-N-carbonyle ou 4-méthanylylidène-pipéridinocarbonyle, auquel cas le groupe -D-E est lié au groupe méthanylylidène, un groupe 4-hydroxy-4-pipéridinylène-N-carbonyle, 4-carboxyméthyl-4-pipéridinylène-N-carbonyle ou 4-méthoxycarbonyl-méthyle-4-pipéridinylène-N-carbonyle, auquel cas le groupe -D-E est lié à la position 4, ou un groupe [[[2-(méthoxyphényl)-éthyl]-amino-carbonyl]-méthylène]-aminocarbonyle, auquel cas le groupe -D-E est lié à l'atome de carbone du méthylène,

D représente une liaison, un groupe alkylène linéaire ou ramifié de 1 à 3 atomes de carbone ou un groupe alkylènephénylène de 1 ou 2 atomes de carbone dans la partie alkylène et

E représente un groupe carboxyle, sulfo, phosphono, 5-tétrazolyle ou O-méthyl-phosphono, un groupe alcoxycarbonyle ayant au total 2 à 5 atomes de carbone dans lequel la partie alcoxy peut être substituée en position 1 ou 2 par un groupe phényle, un groupe aminocarbonyle, méthylaminocarbonyle ou diméthylaminocarbonyle, auquel cas au moins l'un des restes A, B, C ou D ne représente pas une liaison, le reste E ne peut pas suivre immédiatement un hétéroatome des restes A ou C et, dans la mesure où X représente un groupe aminométhyle, la plus courte distance entre le groupe amino et le reste E est d'au moins 12 liaisons,

leurs stéréoisomères, y compris leurs mélanges et leurs sels.

**2.** Dérivés biphényliques de formule générale I selon la revendication 1, dans lesquels

le cycle phényle lié au reste X n'est pas substitué et le cycle phényle lié au reste A peut être substitué par un groupe hydroxyle ou méthoxy,

X représente un groupe aminométhyle ou amidino dans lesquels un atome d'hydrogène sur l'un des atomes d'azote peut être remplacé par un groupe méthoxycarbonyle, éthoxycarbonyle ou benzyloxycarbonyle,

A représente une liaison, un atome d'oxygène, un groupe -NH-CO-, -CO-NH-, -CO-$NCH_3$-, -$SO_2$-NH- ou -NH-$SO_2$-NH-,

B représente une liaison, un groupe alkylène linéaire de 1 à 5 atomes de carbone, un groupe cyclohexylène ou phénylène,

C représente une liaison ou, dans la mesure où C ne suit pas immédiatement un atome d'oxygène ou un groupe -CO-NH- ou -CO-$NCH_3$- du reste A, un groupe -CO-NH-, un groupe pipéridinylène-N-carbonyle qui est lié en position 3 ou 4 au groupe -D-E, un groupe 4-pipérazinylène-N-carbonyle ou 4-méthanylylidène-pipéridinocarbonyle, auquel cas le groupe -D-E est lié au groupe méthanylylidène, un groupe 4-hydroxy-4-pipéridinylène-N-carbonyle, 4-carboxyméthyl-4-pipéridinylène-N-carbonyle ou 4-méthoxycarbonylméthylène-pipéridinylène-N-carbonyle, auquel cas le reste -D-E est lié à la position 4, ou un groupe [[[2-(4-méthoxyphényl)-éthyl]-aminocarbonyl]-méthylène]-aminocarbonyle, auquel cas le groupe -D-E est lié à l'atome de carbone du méthylène,

D représente une liaison, un groupe méthylène, éthylène, méthylènephénylène ou éthylènephénylène et

E représente un groupe carboxyle, méthoxycarbonyle, éthoxycarbonyle, benzyloxycarbonyle, aminocarbonyle, diméthylaminocarbonyle ou 5-tétrazolyle, auquel cas au moins l'un des restes A, B, C ou D ne représente pas une liaison et E ne peut pas suivre immédiatement un hétéroatome des restes A

ou C, et, dans la mesure où X représente un groupe aminométhyle, la plus courte distance entre le groupe amino et le reste E est d'au moins 12 liaisons,
leurs stéréoisomères, y compris leurs mélanges et leurs sels.

**3.** Dérivés biphényliques de formule générale I selon la revendication 1, dans lesquels
le reste biphénylyle n'est pas substitué,
X représente un groupe aminométhyle ou amidino, dans lesquels un atome d'hydrogène sur l'un des atomes d'azote peut être remplacé par un groupe méthoxycarbonyle ou benzyloxycarbonyle,
A représente une liaison, un atome d'oxygène, un groupe -NH-CO- ou -CO-NH-,
B représente une liaison, un groupe alkylène linéaire de 1 à 5 atomes de carbone ou un groupe cyclohexylène,
C représente une liaison ou, dans la mesure où C ne suit pas immédiatement un hétéroatome ou un groupe carbonyle du reste A, un groupe -CO-NH-, un groupe pipéridinylène-N-carbonyle qui est lié en position 3 ou 4 au groupe -D-E, un groupe 4-pipérazinylène-N-carbonyle, auquel cas le reste -D-E est lié à la position 4, ou un groupe [[[2-(4-méthoxyphényl)-éthyl]-aminocarbonyl]-méthylène]-aminocarbonyle, auquel cas le groupe -D-E est lié à l'atome de carbone du méthylène,
D représente une liaison, un groupe méthylène ou éthylène et
E représente un groupe carboxyle, méthoxycarbonyle, éthoxycarbonyle ou benzyloxycarbonyle, auquel cas au moins l'un des restes A, B, C ou D ne représente pas une liaison et E ne peut pas suivre immédiatement un hétéroatome des restes A ou C, et, dans la mesure où X représente un groupe aminométhyle, la plus courte distance entre le groupe amino et le reste E est d'au moins 12 liaisons,
leurs stéréoisomères, y compris leurs mélanges et leurs sels.

**4.** Composés suivants de formule générale I selon la revendication 1:
4-amidino-4'-[(4-carboxyméthyl-pipéridino)-carbonyl]-biphényle,
4-amidino-4'-[(4-carboxyméthyl-pipérazino)-carbonyl]-biphényle,
4-amidino-4'-[(4-carboxy-cyclohexyl)-aminocarbonyl]-biphényle,
4-amidino-4'-[(4-méthoxycarbonylméthyl-piperidino)-carbonyl]-biphényle,
4-amidino-4'-[(4-méthoxycarbonyl-cyclohexyl)-aminocarbonyl]-biphényle,
4-(N-méthoxycarbonyl-amidino)-4'-[(4-méthoxycarbonylméthyl-pipéridino)-carbonyl]-biphényle,
4-amidino-3'-[(4-carboxy-cyclohexyl)-aminocarbonyl]-4'-méthoxy-biphényle,
4-aminométhyl-3'-[(4-carboxy-cyclohexyl)-aminocarbonyl]-4'-méthoxy-biphényle et
4-amidino-3'-[N-(4-carboxy-cyclohexyl)-N-méthyl-aminocarbonyl]-4'-hydroxy-biphényle,
leurs stéréoisomères, y compris leurs mélanges et leurs sels.

**5.** Sels d'addition physiologiquement acceptables des composés selon au moins l'une des revendications 1 à 4 avec des acides ou des bases inorganiques ou organiques.

**6.** Médicament contenant un composé selon au moins l'une des revendications 1 à 4 ou un sel d'addition physiologiquement acceptable selon la revendication 5 et éventuellement un ou plusieurs supports et/ou diluants inertes.

**7.** Utilisation d'un composé selon au moins l'une des revendications 1 à 5 pour la préparation d'un médicament qui convient pour la lutte contre ou la prévention des maladies dans lesquelles il apparaît des agrégats de cellules de taille relativement petite ou relativement grande ou dans lesquelles des interactions cellule-matrice jouent un rôle.

**8.** Procédé de préparation d'un médicament selon la revendication 6, caractérisé en ce qu'un composé selon au moins l'une des revendications 1 à 5 est incorporé par voie non chimique dans un ou plusieurs supports et/ou diluants inertes.

**9.** Procédé de préparation des dérivés biphényliques selon les revendications 1 à 5, caractérisé en ce que
a) pour la préparation de composés de formule générale I dans laquelle X représente un groupe amidino éventuellement substitué par un groupe alkyle de 1 à 4 atomes de carbone, un composé éventuellement formé dans le mélange réactionnel, de formule générale

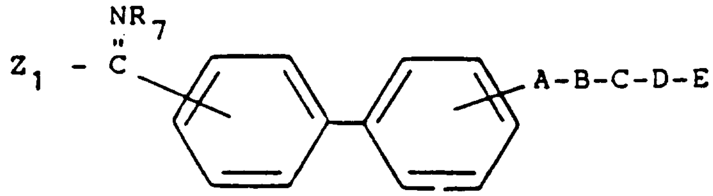

(II)

dans laquelle

A, B, C, D et E sont définis comme dans les revendications 1 à 4,

$R_7$ représente un atome d'hydrogène ou un groupe alkyle de 1 à 4 atomes de carbone et

$Z_1$ représente un groupe alcoxy, aralcoxy, alkylthio, aralkylthio ou amino, est mis à réagir avec une amine de formule générale

$NH_2$ - $R_8$ (III)

dans laquelle

$R_8$ représente un atome d'hydrogène ou un groupe alkyle de 1 à 4 atomes de carbone, ou avec ses sels d'addition d'acide ou bien

b) pour la préparation de composés de formule générale I dans laquelle X représente un groupe aminométhylène, un composé de formule générale

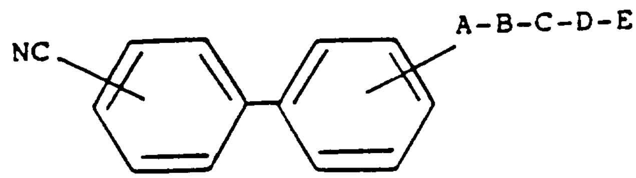

(IV)

dans laquelle

A, B, C, D et E sont définis comme dans les revendications 1 à 4, est réduit ou bien

c) pour la préparation de composés de formule générale I dans laquelle X représente un groupe guanidino, un composé de formule générale

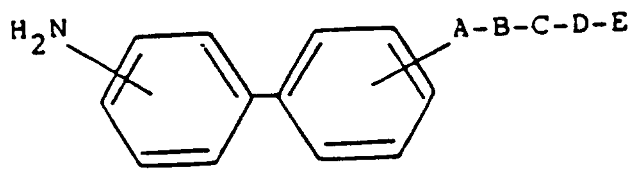

(V)

dans laquelle

A, B, C, D et E sont définis comme dans les revendications 1 à 4, ou son sel d'addition d'acide, est mis à réagir avec le cyanamide ou bien

d) pour la préparation de composés de formule générale I dans laquelle E représente un groupe carboxyle, un composé de formule générale

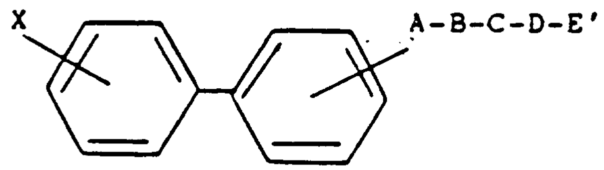

(VI)

dans laquelle

A, B, C, D et X sont définis comme dans les revendications 1 à 4 et

E', qui est lié à un atome de carbone, représente un groupe qui peut être converti en un groupe carboxyle ou bis(hydroxycarbonyl)méthyle par hydrolyse, traitement avec des acides, thermolyse ou hydrogénolyse, est converti en un composé correspondant puis, si nécessaire, un composé bis-(hydroxycarbonyl)méthyle ainsi obtenu est décarboxylé ou bien

e) pour la préparation de composés de formule I dans laquelle A représente un groupe -NH-CO-, $-NCH_3-CO-$, -CO-NH-, $-CO-NCH_3-$, $-SO_2-NH-$ ou $-SO_2-NCH_3-$, un composé de formule générale

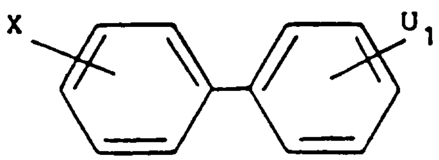

(VII)

est mis à réagir avec un composé de formule générale

$U_2$ - C - D - E (VIII)

dans lequelles

C, D, E et X sont définis comme dans les revendications 1 à 4, l'un des restes $U_1$ ou $U_2$ représente un groupe $HNR_3$ où $R_3$ représente un atome d'hydrogène ou un groupe méthyle, et

l'autre des restes $U_1$ ou $U_2$ représente un groupe $Z_2$-A' où

A' représente un groupe carbonyle ou sulfonyle et

$Z_2$ représente un groupe hydroxyle ou un groupe partant nucléophile,

ou avec ses dérivés réactifs, ou bien

f) pour la préparation de composés de formule générale I dans laquelle le groupe E-D-C représente un groupe $HOOC-(CH_2)_n-CO-NH$, un composé de formule générale

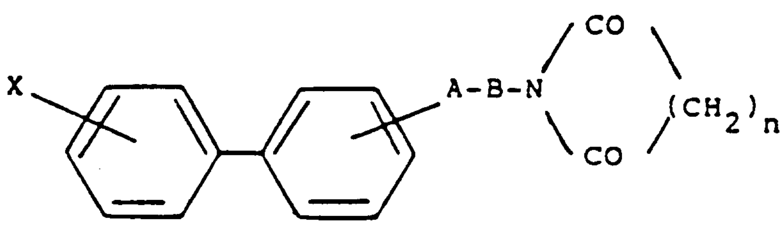

(IX)

dans laquelle

A, B, X et n sont définis comme dans les revendications 1 à 4, est hydrolysé ou bien

g) pour la préparation de composés de formule générale I dans laquelle E représente un groupe alcoxycarbonyle ayant au total 2 à 5 atomes de carbone, dans lequel la partie alcoxy peut être substituée en position 1 ou 2 par un groupe phényle, un composé de formule générale

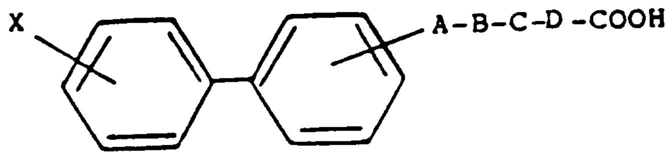

(X)

dans laquelle

A, B, C, D et X sont définis comme dans les revendications 1 à 4, ou ses dérivés réactifs, est mis à réagir avec un composé de formule générale

$H - R_9$ (XI)

dans laquelle

$R_9$ représente un groupe alcoxy de 1 à 4 atomes de carbone dans lequel la partie alcoxy peut être substituée en position 1 ou 2 par un groupe phényle, ou bien

h) pour la préparation de composés de formule générale I dans laquelle le groupe X contient un groupe méthoxycarbonyle, éthoxycarbonyle ou benzyloxycarbonyle, un composé de formule générale

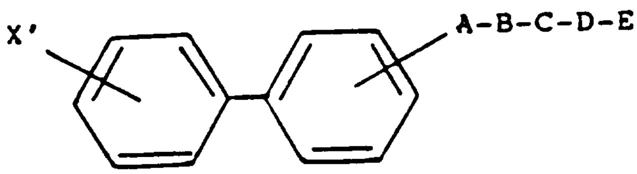

(XII)

dans laquelle

A, B, C, D et E sont définis comme dans les revendications 1 à 4 et

X' représente un groupe aminométhyle, amidino ou guanidino, est mis à réagir avec un ester de formule générale

$Z_3 - CO - OR_3'$ (XIII)

dans laquelle

$R_3'$ représente un groupe méthyle, éthyle ou benzyle et

$Z_3$ représente un groupe partant nucléophile, ou bien

i) pour la préparation de composés de formule générale I dans laquelle A ou C contient un groupe sulfinyle ou sulfonyle ou le cycle phényle lié au reste A contient un groupe méthylsulfinyle ou méthylsulfonyle, un composé de formule générale

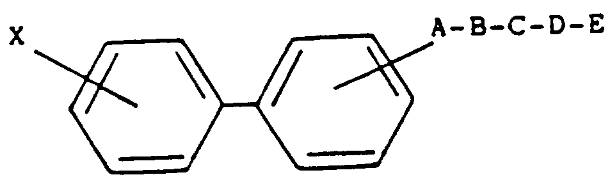

(XIV)

dans laquelle

A, B, C, D, E et X sont définis comme dans les revendications 1 à 4 à condition que A ou C contienne un atome de soufre ou un groupe sulfinyle ou que le cycle phényle lié au reste A

contienne un groupe méthylsulfényle ou méthylsulfinyle et

si nécessaire un reste protecteur utilisé pendant les réactions a) à i) pour la protection de groupes réactifs est clivé et/ou

si on le souhaite un composé de formule générale I ainsi obtenu est résolu en ses isomères cis/trans, en ses énantiomères et/ou diastéréoisomères, et/ou

un composé de formule générale I ainsi obtenu est converti en ses sels, en particulier pour l'utilisation pharmaceutique en ses sels physiologiqment acceptables avec un acide ou une base inorganique ou organique.